# EUROPEAN PATENT APPLICATION

(11) **EP 4 597 073 A1**
(43) Date of publication of application: **06.08.2025**
(21) Application number: 25154497.9
(22) Date of filing: 28.01.2025
(51) Int. Cl.: G01N 15/10, G01N 33/543

(54) **CELL VIABILITY COMPENSATION (CVC) BEADS FOR FLOW CYTOMETRY APPLICATIONS AND METHODS OF USING THE SAME**

(30) Priority: 31.01.2024 US 202463627756 P
(71) Applicant: Becton, Dickinson and Company, Franklin Lakes, NJ 07417-1880 (US)
(72) Inventor: Fan, WEI, Franklin Lakes 07417-1880 (US); SHI, Shirley, Franklin Lakes 07417-1880 (US); MEHRPOUYAN, Majid, Franklin Lakes 07417-1880 (US); GURYEV, Oleg, Franklin Lakes 07417-1880 (US); JENNINGS, Travis Leon, Franklin Lakes 07417-1880 (US)
(74) Representative: Arnold & Siedsma

(57) **Abstract**

The present disclosure provides control compositions as well as methods of making and using the same. Aspects of the control compositions include a control particle characterized by: a core; an aminated polymer including an amine configured to react with an amine reactive dye; and a nucleic acid configured to bind to a DNA binding dye, wherein the aminated polymer and the nucleic acid are covalently associated with the core. Aspects of the invention further include methods of synthesizing the control particles of the compositions, the synthesis methods including: obtaining a core; and covalently associating a polymer including an amine and a nucleic acid with the core to produce a control particle. Also provided are methods for using the control compositions to perform compensation on cell viability data obtained using, e.g., flow cytometry, as well as kits for practicing the subject methods.

## Description

This application claims priority to the filing date of United States Provisional Patent Application Serial No. 63/627,756 filed on January 31, 2024.

### INTRODUCTION

Light detection is often used to characterize components of a biological sample. When a sample is irradiated, light may be scattered, transmitted through, or emitted by the sample (e.g., by fluorescence). Variations in sample components such as morphologies, absorptivity, and the presence of fluorescent labels may cause variations in the light that is scattered, transmitted, or emitted by the sample. To quantify these variations, the light is collected and directed to the surface of a detector. One technique that utilizes light detection to characterize the components of a sample is flow cytometry.

In flow cytometry applications, viability staining has been used to differentiate viable from non-viable mammalian cells based on fluorescence intensity. Viability staining works due to differences between the cell membranes of necrotic (i.e., non-viable) cells and healthy (i.e., viable) cells. Within necrotic cells, compromised cell membranes allow dyes to enter cell interiors and interact with various intracellular components, whereas the intact membranes of healthy cells prevent the majority of dyes from gaining such access.

There are, at present, two general techniques primarily employed for viability staining: staining assays based on amine-reactive fluorescent dyes, and staining assays based on DNA-intercalating dyes. For amine-reactive dye-based assays, the reactive dye can enter the membranes of non-viable cells and react with large quantities of free amines on interior proteins, resulting in intense fluorescent labeling of the non-viable cells of a sample (i.e., relative to viable cells). Similarly, for DNA-intercalating dye-based assays, the DNA binding dye can enter through membranes of non-viable cells and react with DNA in the nucleus, resulting in relatively intense fluorescent labeling of non-viable cells.

Like other sample characterization techniques using flow cytometry, viability staining assays require controls in order to, e.g., set voltage and compensation parameters and obtain accurate and reproducible results. Unfortunately, the majority of current viability staining protocols require a portion of an experimenter's often limited cellular sample to be depleted in order to create the necessary viability controls. Further, the creation of such controls is often time consuming and unreliable, failing to achieve the required brightness separation between live and dead cells needed to attain meaningful results.

### SUMMARY

Thus, there is a need for improved and useful control compositions for cell viability assays. Embodiments of this invention provide such new and useful control compositions, as well as methods of use thereof, addressing the limitations mentioned above. To accomplish this, the invention embodiments leverage recent advances in materials science and biochemistry, along with novel particle coating techniques, to efficiently synthesize a new variety of convenient and effective control particle. The coating techniques of the present disclosure utilize a unique layer-by-layer approach to allow for the creation of highly versatile compensation particles capable of binding both amine-reactive and DNA-intercalating dyes. This versatility, coupled with the highly adjustable nature of the disclosed particle synthesis methods, enables the simplification of current laboratory workflows while also granting users added flexibility when developing future experimental designs. Accordingly, embodiments of the disclosed compositions and methods, e.g., as described in greater detail below, find use in a variety of applications where it is desirable to accurately and adaptively perform cell viability assays with enhanced efficiency at a low cost.

In one aspect, control particles for performing cell viability assays are provided. Aspects of the control particles include: a core; a polymer including an amine configured to react with an amine reactive dye (i.e., an aminated polymer); and a nucleic acid configured to bind to a DNA binding dye, wherein the aminated polymer and the nucleic acid are covalently associated with the core.

In certain embodiments, the core of the control particle has substantially no autofluorescence. In some embodiments, the core has low non-specific binding. In some embodiments, the core includes a polymer such as, e.g., poly(methyl methacrylate) (PMMA) or a polyacrylamide hydrogel. In some embodiments, the core includes an inorganic material such as, e.g., silicon dioxide (i.e., silica). In some embodiments, the diameter of the core may range from 1 µm to 10 µm. In some embodiments, the nucleic acid is covalently bonded to a surface of the core either directly or through a linker and the aminated polymer is covalently bonded to the nucleic acid. In some embodiments, the aminated polymer and the nucleic acid are layered on the core. In some embodiments, a plurality of aminated polymer layers of coating and/or nucleic acid layers of coating are covalently associated with the core. In some embodiments, the particle includes 5 or more aminated polymer layers and/or 5 or more nucleic acid layers. In some embodiments, the aminated polymer layers of coating alternate with the nucleic acid layers of coating. In some embodiments, the control particle is a bead.

In certain embodiments, the one or more aminated polymers of the control particle each include multiple amines. In some embodiments, each aminated polymer includes a spacing between each amine sufficient to prevent self-quenching of the amine reactive dye. In some embodiments, the one or more aminated polymers include a polypeptide such as, e.g., a protein. In some embodiments, the protein includes arginine and/or lysine residues. In some embodiments, the protein is a histone or a myelin basic protein (MBP). In some embodiments, the one or more aminated polymers include a polysaccharide such as, e.g., a cationic polymer. In some embodiments, the cationic polymer includes one or more of: branched polyethylenimine, polyallylamine, polylysine, or chitosan. In some embodiments, the one or more amines of each aminated polymer are configured to react with an amine reactive dye free flowing in a solution. In some embodiments, the amine reactive dye has an emission maximum wavelength ranging from 400 nm to 850 nm. In some embodiments, the amine reactive dye has an absorption maximum (excitation maximum) ranging from 300 nm and 800 nm. In some embodiments, the amine reactive dye includes a BD Horizon^{™} reagent such as, e.g., a BD Horizon^{™} Fixable Viability Stain (FVS) reagent.

In certain embodiments, the one or more nucleic acids of the control particle have a size ranging from 300 base pairs to 700 base pairs. In some embodiments, the one or more nucleic acids of the control particle each include deoxyribonucleic acid (DNA). In some embodiments, the DNA includes double stranded DNA (dsDNA). In some embodiments, the DNA includes naturally occurring DNA sequences such as, e.g., DNA sequences obtained from salmon. In some embodiments, the DNA includes synthetic DNA sequences. In some embodiments, the one or more nucleic acids are configured to bind to a DNA binding dye free flowing in a solution. In some embodiments, the DNA binding dye has an emission maximum wavelength ranging from 400 nm to 850 nm. In some embodiments, the DNA binding dye has an absorption maximum (excitation maximum) ranging from 300 nm and 800 nm. In some embodiments, the DNA binding dye includes propidium iodide (PI), 7-Amino-Actinomycin D (7-AAD) and/or 4',6-diaminidino-2-phenylindole (DAPI).

In certain embodiments, the control particle is a positive control particle. In some embodiments, the positive control particle is labeled with a plurality of amine reactive dyes covalently bound to the amines of the one or more aminated polymers and/or a plurality of DNA binding dyes bound to the one or more nucleic acids. In certain embodiments, the control particle is a negative control particle. In some embodiments, the negative control particle includes one or more polyethylene glycol (PEG) compounds configured to prevent an amine reactive dye from reacting with an amine of the negative control particle. In some embodiments, the negative control particle does not include the aminated polymer or the nucleic acid, e.g., the negative control particle consists of a core.

In certain embodiments, a control composition is provided including a plurality of control particles. In some embodiments, the control composition includes a plurality of unlabeled positive control particles. In some embodiments, the unlabeled control composition further includes a plurality of negative control particles. In some embodiments, the control composition includes a plurality of labeled positive control particles. In some embodiments, the labeled control composition further includes a plurality of negative control particles. In some embodiments, the control composition includes a liquid solution such as, e.g., a buffer. In some embodiments, the control composition is lyophilized.

In another aspect, methods of synthesizing the control particles of the invention are provided. Aspects of the methods include: obtaining a core; and covalently associating a polymer including an amine and a nucleic acid with the core to produce a control particle.

In certain embodiments, the surface of the core includes a functional group such as, e.g., an amide, a maleimide, or a thiol group. In some embodiments, the method further includes surface functionalization of the core with the functional group. For example, the obtained core may include silica and the functionalization may include amination. In some embodiments, the method further includes modifying the functional group of the core to include a reactive functional group using, e.g., a reagent. For example, an aminated silica core may be modified to include a thiol group using a thiolating reagent such as, e.g., 2-iminothiolane. In some embodiments, the method further includes positively charging the core, e.g., to increase efficiency in binding with the nucleic acid prior to associating the aminated polymer with the core. In some embodiments, the method further includes negatively charging the core, e.g., increase efficiency in binding with the aminated polymer prior to associating the nucleic acid with the core.

In certain embodiments, the nucleic acid includes deoxyribonucleic acid (DNA) such as, e.g., double stranded DNA (dsDNA). In some embodiments, the particle synthesis method further includes modifying the DNA to include a functional group reactive to a functional group of the core and/or a functional group of the aminated polymer. For example, the core and/or the aminated polymer may be modified to include a thiol group and the DNA may be modified to include a maleimide group. In some embodiments, the nucleic acid is covalently associated with the core by reacting the functional group of the DNA with the reactive functional group of the core. In some embodiments, the nucleic acid is covalently associated with the core by reacting the functional group of the DNA with a functional group of the aminated polymer, e.g., when the aminated polymer is already covalently associated with the core. In some embodiments, the 5' end and/or the 3' end of the DNA is functionalized. In some embodiments, the DNA is dsDNA and both strands of the dsDNA are functionalized. In some embodiments, the dsDNA is covalently bonded to the core by reacting a functional group of a first strand of the dsDNA with the reactive functional group of the core and the dsDNA is covalently bonded to the aminated polymer by reacting a functional group of a second strand of the dsDNA to a functional group of the aminated polymer. In some embodiments, both strands of the dsDNA are covalently bonded to aminated polymers.

In certain embodiments, the particle synthesis methods further includes modifying the aminated polymer to include a functional group reactive to a functional group of the core and/or a functional group of the nucleic acid. For example, the core and/or the nucleic acid may be modified to include a maleimide group and the aminated polymer may be modified to include a thiol group. In some embodiments, the aminated polymer is covalently associated with the core by reacting the functional group of the aminated polymer with the reactive functional group of the core. In some embodiments, the aminated polymer is covalently associated with the core by reacting the functional group of the aminated polymer with a functional group of the nucleic acid, e.g., when the nucleic acid is already covalently associated with the core. In some embodiments, the aminated polymer is functionalized with two or more functional groups. For example, the aminated polymer may be a protein and two or more lysine residues of the protein may thiolated using, e.g., 2-iminothiolane. In some embodiments, the aminated polymer is covalently bonded to the core by reacting a first functional group of the aminated polymer with the reactive functional group of the core and the aminated polymer is covalently bonded to the nucleic acid by reacting a second functional group of the aminated polymer to a functional group of the nucleic acid. In some embodiments, two or more functional groups of the aminated polymer are covalently bonded to nucleic acids.

In certain embodiments, the aminated polymer and the nucleic acid are covalently associated with the core by alternately layering coatings of the aminated polymer and the nucleic acid. For example, the nucleic acid may include dsDNA and two or more of the nucleic acids may be modified to include a maleimide group on the 5' end of each strand of dsDNA, the aminated polymer may include a protein and two or more of the aminated polymers may be modified to include two or more thiol groups, and the core may include a plurality of thiol groups. The layering may then include reacting the maleimide of a first 5' end of a first nucleic acid with a thiol group of the core, reacting the maleimide of a second 5' end of the first nucleic acid with a first thiol group of a first aminated polymer, reacting a second thiol group of the first aminated polymer with the maleimide of a first 5' end of a second nucleic acid, reacting the maleimide of a second 5' end of the second nucleic acid with a first thiol group of a second aminated polymer, etc. In some embodiments, 5 or more rounds of layering are performed such that the control particle includes 5 or more nucleic acid layers and 5 or more aminated polymer layers. In some embodiments, each round of layering includes covalently associating a plurality of nucleic acids and a plurality of aminated polymers to the core.

In certain embodiments, the control particle is a positive control particle. In some embodiments, the particle synthesis method further includes incubating the positive control particle in a solution containing a plurality of amine reactive dyes and/or DNA binding dyes in order to create a labeled positive control particle. In certain embodiments, the control particle is a negative control particle. In some embodiments, the particle synthesis method further includes conjugating one or more polyethylene glycol (PEG) compounds to an amine of the negative control particle. In some embodiments, the negative control particle does not include the aminated polymer or the nucleic acid. In some embodiments, synthesizing the negative control particle includes conjugating one or more polyethylene glycol (PEG) compounds to one or more amine groups of the core in order to prevent an amine reactive dye from reacting with the core.

In another aspect, methods of determining a compensation value for cell viability data obtained from a particle analyzer (e.g., a flow cytometer) using the control particles of the invention are provided. Aspects of the methods include: analyzing a labeled compensation control composition using a flow cytometer to obtain flow cytometry data; and calculating a compensation value based on the flow cytometry data.

In certain embodiments, the compensation value determination methods further include preparing the labeled compensation control composition. In some embodiments, the preparing includes contacting an unlabeled compensation control composition with an amine reactive dye and/or a DNA binding dye. In certain embodiments, the determined compensation value is used to perform compensation on cell viability data. In some embodiments, the compensation is performed by: contacting a cell sample with the same dye that is bound to the labeled positive control particle; producing the cell viability data by analyzing the cell sample with a particle analyzer (e.g., a flow cytometer); modifying the cell viability data using the determined compensation value.

In another aspect, a kit is provided including a compensation control composition as described herein. In some embodiments, the kit further includes an amine reactive dye and/or a DNA binding dye for labeling the positive control particles of the compensation control composition. In some embodiments, the kit further includes instructions for using the compensation control composition to determine a compensation value for cell viability data obtained from a flow cytometry analysis. In some embodiments, the kit further includes instructions for performing the flow cytometry analysis and using the determined compensation value to perform compensation on the obtained cell viability data.

### BRIEF DESCRIPTION OF THE FIGURES

**FIG. 1** provides an illustration of the mechanisms for two types of viability staining in accordance with an embodiment of the invention.
**FIG. 2** depicts a representation of a cell viability compensation (CVC) bead in accordance with an embodiment of the invention.
**FIG. 3** illustrates an exemplary method for synthesizing CVC beads in accordance with an embodiment of the invention.
**FIG. 4** provides staining results for prior art compensation beads and CVC beads synthesized in accordance with an embodiment of the invention for the BD Horizon^{™} FVS520 amine-reactive dye.
**FIG. 5** provides staining results for prior art compensation beads and CVC beads synthesized in accordance with an embodiment of the invention for the BD Horizon^{™} FVS570 amine-reactive dye.
**FIG. 6** provides staining results for prior art compensation beads and CVC beads synthesized in accordance with an embodiment of the invention for the BD Horizon^{™} FVS700 amine-reactive dye.
**FIG. 7** provides staining results for prior art compensation beads and CVC beads synthesized in accordance with an embodiment of the invention for two DNA-intercalating dyes, BD Pharmingen^{™} 7-AAD and BD Pharmingen^{™} DAPI.
**FIG. 8** provides the results of a stability study performed for CVC beads synthesized in accordance with an embodiment of the invention.
**FIGS. 9A** to **9B** provide schematics of exemplary particle sorting systems for use with the CVC beads of the invention according to certain embodiments.
**FIG. 10** depicts a functional block diagram of an exemplary particle analyzer control system for use with the CVC beads of the invention according to certain embodiments.
**FIGS. 11A** to **11B** illustrate an exemplary particle analysis system for use with the CVC beads of the invention according to certain embodiments. **FIG. 11A** depicts a functional block diagram of the particle analysis system. **FIG. 11B** depicts a flow cytometer of the particle analysis system.
**FIG. 12A** to **12B** illustrate an exemplary particle sorter and data processing technique for fluorescence imaging using radiofrequency tagged emissions for use in performing viability staining assays according to certain embodiments. **FIG. 12A** provides schematics of the image-enabled particle sorter. **FIG. 12B** provides a flow chart depicting the data processing technique.
**FIG. 13** depicts a block diagram of a computing system for use with the CVC beads of the invention according to certain embodiments.

### DETAILED DESCRIPTION

The present disclosure provides control compositions as well as methods of making and using the same. Aspects of the control compositions include a control particle characterized by: a core; a polymer including an amine configured to react with an amine reactive dye; and a nucleic acid configured to bind to a DNA binding dye, wherein the aminated polymer and the nucleic acid are covalently associated with the core. Aspects of the invention further include methods of synthesizing the control particles of the compositions, the synthesis methods including: obtaining a core; and covalently associating a polymer including an amine and a nucleic acid with the core to produce a control particle. Also provided are methods for using the control compositions to perform compensation on cell viability data obtained using a particle analyzer (e.g., a flow cytometer) as well as kits for practicing the subject methods.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

While the apparatus and method has or will be described for the sake of grammatical fluidity with functional explanations, it is to be expressly understood that the claims, unless expressly formulated under 35 U.S.C. §112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 U.S.C. §112 are to be accorded full statutory equivalents under 35 U.S.C. §112.

As summarized above, control compositions are provided. In further describing various embodiments of the invention, the subject compositions including, e.g., the control particles of the compositions are first described in greater detail. Next, methods of synthesizing the control particles of the compositions are described. In addition, methods of using the control compositions to perform compensation on cell viability data, as well as kits for practicing the subject methods, are also provided.

### CONTROL COMPOSITIONS

As summarized above, control compositions, e.g., flow cytometry compensation control compositions, are provided. The control compositions may be used to validate the functionality or verify the performance of particle analysis systems (e.g., flow cytometry systems), protocols, and reagents for use in flow cytometry assays. In certain embodiments, the control compositions may be used to interpret flow cytometry data.

In some embodiments, the control compositions are used to perform fluorescence compensation for flow cytometry data including, e.g., correcting for fluorescence spillover by removing the signal of a given fluorescent particle (e.g., a given fluorophore or fluorochrome) from each secondary channel or detector. In other words, the signal of a given fluorescent particle is removed from all the detectors or channels of a flow cytometer except the channel/detector specifically designated to measure the fluorescent particle. In some cases, fluorescence compensation may be performed for flow cytometric cell viability assays using the subject control compositions (e.g., cell viability compensation [CVC] beads) in order to, e.g., distinguish between live and dead cell populations in flow cytometry data. In some embodiments, the flow cytometric cell viability assay uses an amine reactive dye to distinguish between live and dead cells. In certain embodiments, the flow cytometric cell viability assay uses a DNA binding dye to distinguish between live and dead cells.

In some embodiments, the control compositions of the invention may be positive control compositions. A positive control composition may include a positive control particle configured to bind to the fluorescent particles (e.g., fluorophores) of a given assay. In some embodiments, the positive control particle is configured to bind to the fluorophores of both amine-reactive fluorescent dye-based cell viability assays and DNA-intercalating dye-based cell viability assays (i.e., the positive control particle is configured to bind to both amine-reactive fluorescent dyes and DNA-intercalating dyes). In some cases, the positive control particle may be configured to bind to other fluorescent particles in addition to the amine-reactive fluorescent dyes and the DNA-intercalating dyes. For example, the positive control particle may be configured to bind to one or more different fluorescently labeled antibodies and/or one or more different fluorescently labeled nucleic acid probes in addition to the amine-reactive fluorescent dyes and the DNA-intercalating dyes.

As described above, embodiments of the control compositions include a positive control particle. The positive control particle may include or be characterized by: a core; a polymer including an amine configured to react with an amine reactive dye (e.g., an aminated polymer); and a nucleic acid configured to bind to a DNA binding dye, wherein the aminated polymer and the nucleic acid are covalently associated with the core. In some embodiments, the core of the positive control particle includes a material with low autofluorescence and/or low non-specific binding. In some embodiments, the core of the positive control particle is substantially non-autofluorescent and/or exhibits low non-specific binding, e.g., when considered as a whole. In some embodiments, the aminated polymer and the nucleic acid molecules are layered on the core. In other words, a plurality of aminated polymer molecules and/or nucleic acid molecules covalently associated with the core may be relatively evenly dispersed across a surface of the core to form a coating on the core. In some cases, the nucleic acid molecules and the aminated polymer molecules form separate, alternating, layers of coatings on the surface of the core. In these instances, the positive control particle may include multiple layers of each of the aminated polymer and the nucleic acid molecules such as, e.g., 2 or more layers of each, or 3 or more, or 4 or more, or 5 or more, or 10 or more, etc. In some cases, the positive control particle includes a number of layers sufficient to bind an amount of an amine reactive dye and/or a DNA binding dye (e.g., of a given cell viability assay) such that the positive control particle is at least as bright as a cell stained using the same dye. In these instances, the positive control particle may include a number of layers sufficient to bind an equal or greater amount of the amine reactive dye and/or the DNA binding dye as a non-viable cell of a specific cell viability assay (e.g., a non-viable mammalian tissue cell grown under specific conditions). In some embodiments, the positive control particle may be configured to have substantially the same autofluorescence as the cells of a given flow cytometry assay (e.g., cell viability assay). In these cases, the cells of interest may be mammalian cells. In some cases, the positive control particle is a bead.

In certain embodiments, the control compositions of the present disclosure may include a negative control particle. In some cases, the negative control particle includes: a core. In some cases, the core of the negative control particle may be substantially the same as the core of the positive control particle. For example, the core of the negative control particle may include the same material(s), be the same size and/or shape, have the same properties (e.g., autofluorescent properties), etc. as the core of the positive control particle. In some embodiments, the core of the negative control particle includes a material with low autofluorescence and/or low non-specific binding. In some embodiments, the core of the negative control particle is substantially non-autofluorescent and/or exhibits low non-specific binding, e.g., when considered as a whole. In certain embodiments, the negative control particle does not include an aminated polymer or a nucleic acid (i.e., does not include any aminated polymer molecules or any nucleic acid molecules). In these instances, the core of the negative control particle may be conjugated with molecules (e.g., hydrophilic polymers such as polyethylene glycol [PEG]) in order to consume surface groups that may react with amine reactive dyes or DNA binding dyes. In some embodiments, a low non-specific binding polymer layer or coating such as, e.g., a silane layer or a layer of hydrophilic polymers (e.g., PEG, poly(vinyl pyrrolidone) (PVP), poly(acrylic acid) (PAA), etc.), may be deposited on the surface of the core of the negative control particle. In some embodiments, the negative control particle is configured to have substantially the same autofluorescence as the positive control particle. In some embodiments, the negative control particle is configured to have substantially the same autofluorescence as the cells of a given flow cytometry assay (e.g., cell viability assay). In these cases, the cells of interest may be mammalian cells. In some cases, the negative control particle is a bead.

The control compositions (e.g., CVC compositions) may include a plurality of positive control particles. In some embodiments, the control compositions may include a plurality of negative control particles. In some cases, the control compositions may include a plurality of both positive and negative control particles. In certain embodiments, the control compositions may include positive control particles labeled with (e.g., bound to/associated with) an amine reactive dye and/or a DNA binding dye. In some embodiments, the control compositions may be suspended in solution (e.g., a liquid buffer). In other cases, the control compositions may include substantially no liquid (e.g., the control compositions may be dehydrated or lyophilized).

In certain embodiments, the control compositions of the present disclosure are storage stable control compositions, where the control compositions are substantially stable for an extended period of time. By "stable" or "storage stable" or "substantially stable" is meant a control composition including control particles that retain their properties (e.g., autofluorescence, binding properties, etc.) and do not significantly degrade/change reactivity over an extended period of time. For example, a stable positive control particle may retain its ability to react to amine reactive dyes and/or bind to DNA binding dyes and a negative control particle may retain its inertness with regards to amine reactive dyes and/or DNA binding dyes, regardless of whether or not, e.g., the negative control particle and the positive control particle are stored together. For example, a storage stable control composition may not have a significant change of dye binding activity (of either the negative or positive control particles of the composition) due to the degradation of the control composition over an extended period of time. This stability may be reflected by having no change or a negligible change in the fluorescence of the positive and/or negative control particles of a control composition resulting from staining with a specific fluorescence particle (e.g., an amine reactive dye or DNA binding dye) such as 10% or less change of fluorescence activity, or 9% or less, or 8% or less, or 7% or less, or 6% or less, or 5% or less, or 4% or less, or 3% or less, or 2% or less, or 1% or less change of fluorescence activity over an extended period of time. In certain instances, a storage stable control composition has 5% or less change of fluorescence activity (e.g., dye binding activity) over an extended period of time.

In some cases, a storage stable control composition substantially retains its fluorescent particle (e.g., amine reactive dye and/or DNA binding dye) binding activities over an extended period of time, such as retains 100% of its activity, or 99% or more, or 98% or more, or 97% or more, or 96% or more, or 95% or more, or 94% or more, or 93% or more, or 92% or more, or 91% or more, or 90% or more, or 85% or more, or 80% or more, or 75% or more of its activity over an extended period of time. For example, a storage stable control composition may retain 90% or more of its binding activity over an extended period of time. In some cases, a storage stable composition retains 95% or more of its binding activity over an extended period of time. An extended period of time is a period of time such as 1 week or more, or 2 weeks or more, or 3 weeks or more, or 1 month or more, or 2 months or more, or 3 months or more, or 4 months or more, or 6 months or more, or 9 months or more, or 1 year or more, or 1.5 years (e.g., 18 months) or more, or 2 years or more, or 2.5 years (e.g., 30 months) or more, or 3 years or more, or 3.5 years (e.g., 42 months) or more, or 4 years or more, or 4.5 years (e.g., 54 months) or more, or 5 years or more. For instance, an extended period of time may be 6 months or more. In some cases, an extended period of time is 9 months or more. In some cases, an extended period of time is 1 year (e.g., 12 months) or more. In some cases, an extended period of time is 1.5 years (e.g., 18 months) or more. In some cases, an extended period of time is 2 years (e.g., 24 months) or more. In some instances, the extended period of time is 10 years or less, such as 7.5 years or less, including 5 years or less, e.g., 2 years or less.

### Positive Control Particles

Embodiments of the control composition include one or more positive control particles. By "positive control particle" is meant a particle, e.g., a bead, configured to bind to an amine binding fluorescent particle and/or a nucleic acid binding fluorescent particle. By "fluorescent particle" is meant a particle including a component that absorbs light or other electromagnetic radiation and subsequently releases light (e.g., a component that absorbs light at a given wavelength and subsequently releases light of a longer wavelength as a result) such as, e.g., a fluorophore or a quantum dot. The amine binding fluorescent particle and the nucleic acid binding fluorescent particle may be an amine reactive dye and a DNA intercalating dye used in flow cytometric cell viability assays, respectively.

As described above, flow cytometric cell viability assays include fluorescent amine reactive dyes and/or DNA intercalating dyes that are used to stain a sample containing cells. In non-viable cells (i.e., dead or dying cells), a compromised cell membrane allows the dye to enter into the cell and interact with cell components, resulting in intense fluorescent labeling. Amine reactive dyes interact with free amines in the interior of a cell, while DNA binding dyes penetrate into the nucleus and bind with double stranded DNA (dsDNA) (e.g., between adjacent base pairs or within minor or major grooves). In contrast, the intact cell membranes of viable cells (i.e., live/healthy cells) do not allow the dyes to access the interior of the cell, resulting in relatively dim staining of viable cells. In some cases, fluorescence intensity is measured using a particle analyzer (e.g., a flow cytometer).

In some embodiments, control compositions (e.g., CVC compositions) including positive control particles of the present disclosure are used to determine a compensation or spillover value for flow cytometric cell viability assays. In flow cytometry, compensation is performed to correct for fluorescence spillover, e.g., the additional detection of a fluorescent signal (e.g., the emission of a specific fluorophore) in a detection channel other than the detection channel specifically designated to measure the fluorescent signal. The range of wavelengths of a given detection channel may be determined by the emission wavelength or wavelengths of the fluorophore to be detected, e.g., the fluorophore of the amine reactive dye or the DNA binding dye. To correct for fluorescence spillover, a positive control particle of the present disclosure may be used to determine the amount of fluorescence spillover produced by a given fluorescent particle, e.g., a given cell viability dye. In these instances, the positive control particles of the control composition may be contacted with one or more cell viability dyes (i.e., one or more amine reactive dyes and/or DNA binding dye) in order to label the positive control particles. The labeled control composition (i.e., including labeled positive control particles) may then be introduced into a flow cytometer in order to measure its fluorescence at one or more excitation wavelengths. A spillover or compensation value may then be calculated for each fluorescent particle (e.g., cell viability dye) of the cell viability assay using the fluorescence measurements generated from the labeled control composition. In some cases, the spillover or compensation values may be calculated by determining the amount of fluorescence detected in secondary channels (i.e., channels other than the detection channel specifically designated to measure a given fluorescent particle) for each fluorescent particle. The compensation/spillover value(s) may then be used to adjust flow cytometry data collected from the labeled sample cells of the cell viability assay.

As described above, positive control particles of the control compositions include: a core; a polymer including an amine configured to react with an amine reactive dye; and a nucleic acid configured to bind to a DNA binding dye, wherein the aminated polymer and the nucleic acid are covalently associated with the core. By "covalently associated" is meant the aminated polymer and the nucleic acid are covalently bound to the surface of the core either directly or through a linker. In some embodiments, the linker may include one or more of the aminated polymer molecules and/or one or more of the nucleic acid molecules. In some embodiments, the aminated polymer and the nucleic acid molecules are layered on the core. In other words, a plurality of aminated polymer molecules and/or nucleic acid molecules covalently associated with the core may be relatively evenly dispersed across a surface of the core to form a coating on the core. In these instances, the positive control particle may include multiple layers of each of the aminated polymer and the nucleic acid such as, e.g., 2 or more layers of each, or 3 or more, or 4 or more, or 5 or more, or 10 or more, etc.

In some instances, the aminated polymer and the nucleic acid molecules are alternately layered on the core. In other words, each layer includes either one or more of the aminated polymer molecules or one or more of the nucleic acid molecules and there are no two consecutive aminated polymer layers or nucleic acid layers. In other cases, the aminated polymer and the nucleic acid are evenly mixed and dispersed across the surface of the core. In some cases, the positive control particle includes a number of layers sufficient to bind an amount of an amine reactive dye and/or a DNA binding dye (e.g., of a given cell viability assay) such that the positive control particle is at least as bright as a cell stained using the same dye (e.g., during a cell viability assay). In these instances, the cell may be a mammalian cell such as. In these instances, the positive control particle may include a number of layers sufficient to bind an equal or greater amount of the amine reactive dye and/or the DNA binding dye as a non-viable cell of a specific cell viability assay (e.g., a non-viable mammalian tissue cell grown under specific conditions). In some embodiments, the positive control particle may be configured to have substantially the same autofluorescence as the cells of a given flow cytometry assay.

As described above, aspects of the control particles of the present disclosure include a core. In some cases, the core of a control particle is substantially non-autofluorescent when exposed to light having a wavelength, e.g., in the range from about 350 nm to about 1000 nm. By "substantially non-autofluorescent" is meant that the core emits less fluorescence than a cell or control particle labeled with a fluorescent particle (e.g., a cell viability dye) when illuminated with a beam of light sufficient to cause a fluorescence emission from the fluorescent particle. In other words, a core of the present disclosure is configured such that fluorescent events resulting from a fluorescent particle (e.g., of a labeled positive control particle or sample cell) are readily and reliably distinguishable from background fluorescence emanating from the core. As such, the inherently emitted fluorescence of the core (i.e., the autofluorescence of the core) does not interfere with accurately determining spillover values for the fluorescent particles used in flow cytometry assays (e.g., spillover values for the cell viability dyes of flow cytometric cell viability assays).

In some embodiments, control compositions of the present disclosure are configured such that, when used to determine the compensation or spillover value(s) for a flow cytometric cell viability assay, the inherent fluorescence of the positive control particles (including, e.g., the inherent fluorescence of the substantially non-autofluorescent core) will not substantially contribute to fluorescence spillover. In some cases, the core includes a low autofluorescence material that emits no fluorescence or substantially no fluorescence. In some embodiments, the core includes a low autofluorescence material that emits no fluorescence or substantially no fluorescence inside of a predetermined range of wavelengths used to calculate the spillover value(s) for the fluorescent particles of a flow cytometry assay of interest (e.g., the cell viability dyes of a cell viability assay). In certain embodiments, the core includes a low autofluorescence material that emits no fluorescence or substantially no fluorescence when excited by ultraviolet (UV) (e.g., from approximately 320nm to 380nm) or violet (e.g., from approximately 390nm to 420nm) light.

In some embodiments, a core of the present disclosure exhibits low non-specific binding. By "low non-specific binding" is meant the core displays low affinity towards (e.g., has a relatively high dissociation constant when interacting with) unwanted biological (e.g., proteins, polysaccharides, nucleic acids, cells, etc.) and non-biological (e.g., silica, cationic polymers, plastics, etc.) materials such that the unwanted materials rarely, if ever, bind to, interact with, or hybridize with the core. In some embodiments, a core of the present disclosure exhibits low non-specific binding towards, and will not substantially interact with, the fluorescent particles (e.g., stains, dyes, labeled antibodies, etc.) of flow cytometry assays (e.g., low non-specific binding towards the cell viability dyes of cell viability assays). For example, the dissociation constant of the interaction between the core and an amine reactive dye may be at least an order of magnitude larger than the dissociation constant of the interaction between the aminated polymer (e.g., as described herein) and the amine reactive dye. In some embodiments, the dissociation constant of the interaction between the core and a DNA binding dye may be at least an order of magnitude larger than the dissociation constant of the interaction between the nucleic acid (e.g., as described herein) and the DNA binding dye. In some embodiments, the core exhibits low non-specific binding towards, and will not substantially interact with, any of the materials used to prepare a sample for flow cytometry analysis.

Cores of the present disclosure may include, but are not limited to, microparticles, beads (e.g., microbeads, magnetic beads, ion torrent beads, flow cytometry beads), or microspheres. A core of the present disclosure may include any material allowing for the core to be substantially non-autofluorescent (e.g., at the excitation wavelengths of the fluorescent particles of flow cytometry assays of interest) and exhibit low non-specific binding (e.g., to fluorescent particles, cells, reagents, and other control particles utilized in flow cytometry assays of interest). In some embodiments, the core may include polymers (e.g., plastic, polydimethylsiloxane [PDMS], polystyrene, polypropylene, agarose, gelatin, hydrogels, methylstyrene, acrylic polymer, latex, sepharose, cellulose, nylon, silicone, poly[methyl methyacrylate] [PMMA], hydrogel, polyacrylamide hydrogel, polysulfone, polyethyl, etc.), ceramics, glass, metals (e.g., titanium, gold, etc.), silica, or any combination thereof. In certain embodiments, the core may consist essentially of silica, PMMA, and/or a polyacrylamide hydrogel. In some embodiments, the core may be a silica bead. In other embodiments, the core may be a PMMA bead or a polyacrylamide hydrogel bead.

The core of the present disclosure may be any suitable size or shape. In some cases, the size of the core is selected to approximately match the size of the cells that are analyzed, e.g., using a flow cytometric cell viability assay. In some embodiments, the diameter of the core is from about 0.1 to about 150 microns, such as, for example, from about 0.1 to 0.5 microns, or from about 0.5 to 1 micron, or from about 1 to 1.5 microns, or from about 1.5 to 5 microns, or from about 5 to 7 microns, or from about 7 to 10 microns, or from about 10 to 25 microns, or from about 25 to 50 microns, or from about 50 to 100 microns, or from about 100 to 150 microns. In certain embodiments, the core is about 6 microns. The shape of the core may be, but is not limited to, spherical, discoidal, ellipsoidal, cubical, cylindrical, pyramidal, irregular or any other suitable shape. In some embodiments, the core is solid. In other embodiments, the core is hollow.

As described above, fluorescent particle binding molecules (e.g., aminated polymer molecules and/or nucleic acid molecules) may be conjugated to the surface of a core of the present disclosure. In some embodiments, the fluorescent particle binding molecules are covalently bound directly to the core. In some cases, the fluorescent particle binding molecules are indirectly covalently bound to the core, e.g., via a linker. In some embodiments, the linker may include one or more of the fluorescent particle binding molecules (e.g., aminated polymer molecules and/or nucleic acid molecules). For example, an aminated polymer molecule may be covalently bound to a nucleic acid molecule, wherein the nucleic acid molecule is covalently bound to the surface of the core either directly or through a linker. In some embodiments, the core may be modified to include chemical groups to facilitate conjugation of the fluorescent particle binding molecules. Suitable modifications and linkers are further described below. In certain embodiments, the fluorescent particle binding molecules conjugated to the surface of the core are configured to bind to cell viability assay dyes such as, e.g., amine reactive dyes or DNA binding dyes and may include the aminated polymers and/or nucleic acids discussed above and further described below. In some embodiments, molecules configured to reduce the binding of unwanted materials (e.g., unwanted biological compounds, non-biological compounds, stains, dyes, antibodies, or other fluorescent or labeling molecules) are conjugated to the surface of the core. Such molecules may include, but are not limited to, e.g., polyethylene glycol (PEG) of various molecular weights and branching structures, zwitterionic polymers, poly(hydroxyfunctional acrylates), poly(2-oxazoline)s, poly(vinylpyrrolidone), poly(glycerol), peptides, proteins, and polysaccharides, polyacrylamide, polyester, , or any combination thereof.

As described above, aspects of the positive control particles of the present disclosure include a core covalently associated with a molecule including an amine configured to react with an amine reactive dye. Any molecule or combination of molecules including an amine (e.g., a primary, secondary, or tertiary amine) that can bind an amine reactive dye may be used in the positive control particles. In some cases, the molecule is an aminated polymer. By "aminated polymer" is simply meant a polymer having one or more amines. In certain embodiments, the one or more aminated polymers of the positive control particle each include multiple amines. In these cases, the aminated polymer(s) may be configured to prevent self-quenching of the amine reactive dye. For example, an aminated polymer may be selected such that its structure does not contribute to self-quenching, e.g., the aminated polymer may include a spacing between each amine sufficient to prevent self-quenching of the amine reactive dye.

In some embodiments, the one or more aminated polymers of a positive control particle include a cationic polymer such as, e.g., a polysaccharide. In some cases, the cationic polymer includes one or more of: branched polyethylenimine, polyallylamine, polylysine, or chitosan. In some embodiments, the aminated polymer includes a polypeptide such as, e.g., a protein. In these cases, the protein may include arginine and/or lysine residues. In some embodiments, the protein is a histone or a myelin basic protein (MBP). In some cases, the protein may be configured to bind to other fluorescent particles in addition to amine reactive dyes. For example, the protein may be configured to bind to a specific binding member conjugated to a fluorophore, wherein the specific binding member may include, e.g., an antibody. As used herein, the term "antibody" is used to refer to a protein consisting of one or more polypeptides substantially encoded by all or part of the recognized immunoglobulin genes. In some cases, the protein may be an epitope of an antigen to which an antibody containing fluorescent particle is configured to bind. In some embodiments, the positive control particle may include two or more different aminated polymers such as, e.g., two or more different proteins. For example, the positive control particle may include 5 layers of aminated polymer molecules, wherein 4 of the aminated polymer layers include histone proteins and the outermost aminated polymer layer includes a CD protein.

In some embodiments, the one or more amines of each aminated polymer are configured to react with an amine reactive dye free flowing in a solution. In some embodiments, the amine reactive dye has an emission maximum wavelength ranging from 350 nm to 900 nm. For example, in some embodiments, the amine reactive dye has an emission maximum wavelength ranging from 400 nm to 900 nm, or 400 nm to 850 nm. In some embodiments, the amine reactive dye has an absorption maximum (excitation maximum) ranging from 300 nm and 1000 nm. For example, in some embodiments, the amine reactive dye has an absorption maximum wavelength ranging from 300 nm to 900 nm, or 300 nm to 800 nm. In some embodiments, the amine reactive dye includes a BD Horizon^{™} reagent such as, e.g., a BD Horizon^{™} Fixable Viability Stain (FVS) reagent. In some cases, the amine reactive dye includes at least one of the dyes selected from the group consisting of: a BD Horizon^{™} Fixable Viability Stain, a Biolegend Zombie^{™} dye, a Thermo Fischer eFluor^{™} dye, a Thermo Fischer LIVE/DEAD^{™} stain, a Proteintech Phantom Dye, and a Tonbo Biosceinces Ghost Dye^{™}.

As described above, aspects of the positive control particles of the present disclosure include a core covalently associated with a nucleic acid configured to bind to a DNA binding dye. Any nucleic acid at any size or combination of nucleic acids (e.g., DNA, ribonucleic acid [RNA], or peptide nucleic acid [PNA]) that can bind a DNA binding dye (e.g., a DNA intercalating dye) may be used in the positive control particles. In certain embodiments, the one or more nucleic acids of a positive control particle have a size ranging from 200 base pairs to 1000 base pairs. In certain embodiments, the one or more nucleic acids of a positive control particle have a size ranging from 300 base pairs to 700 base pairs such as, e.g., 400 base pairs to 600 base pairs including, e.g., 450 base pairs to 550 base pairs. In some embodiments, the one or more nucleic acids of the positive control particle are single stranded. In certain embodiments, the one or more nucleic acids of the positive control particle are double stranded.

In some embodiments, the one or more nucleic acids of the control particle each consist essentially of DNA. In some embodiments, the DNA includes double stranded DNA (dsDNA). In some embodiments, the DNA includes naturally occurring DNA sequences such as, e.g., DNA sequences obtained from salmon. In some embodiments, the DNA includes synthetic DNA sequences. In some cases, the nucleic acid (e.g., dsDNA molecule) may be configured to bind to a DNA intercalating dye. In some embodiments, the nucleic acid may be configured to bind to other fluorescent particles in addition to DNA intercalating dyes. For example, the nucleic acid (e.g., dsDNA molecule) may be configured to bind to a specific binding member conjugated to a fluorophore, wherein the specific binding member may include, e.g., a nucleic acid binding probe. In other words, the nucleic acid may include dsDNA with a sequence configured to bind to a specific gene probe (e.g., a single-stranded DNA or RNA fragment conjugated to a fluorophore). In some embodiments, the positive control particle may include two or more different nucleic acids such as, e.g., two or more dsDNA molecules each including different sequences. For example, the positive control particle may include 5 layers of nucleic acid molecules, wherein 4 of the nucleic acid layers include a dsDNA sequence obtained from salmon and the outermost nucleic acid layer includes a dsDNA sequence substantially the same as all or part of a gene of interest.

In some embodiments, the one or more nucleic acids are configured to bind to a DNA binding dye free flowing in a solution. In some embodiments, the DNA binding dye has an emission maximum wavelength ranging from 350 nm to 900 nm. For example, in some embodiments, the DNA binding dye has an emission maximum wavelength ranging from 400 nm to 900 nm, or 400 nm to 850 nm. In some embodiments, the DNA binding dye has an absorption maximum (excitation maximum) ranging from 300 nm and 1000 nm. For example, in some embodiments, the DNA binding dye has an absorption maximum wavelength ranging from 300 nm to 900 nm, or 300 nm to 800 nm. In some embodiments, the DNA binding dye includes propidium iodide (PI), 7-Amino-Actinomycin D (7-AAD) and/or 4',6-diaminidino-2-phenylindole (DAPI). In some cases, the DNA binding dye includes at least one of the dyes selected from the group consisting of: 7-AAD, DAPI, propidium iodide, a Hoechst dye, ethidium bromide, LDS 751, a Thermo Fischer Sytox^{™} dye, a Thermo Fischer T-PRO^{™} dye, a Thermo Fischer TOTO^{™} dye, a Thermo Fischer YO-PRO^{™} dye, a Biolegend Helix-NP^{™} dye, a Biotium RedDot^{™} dye and a Biostatus Limited DRAQ^{™} dye.

As described above, aspects of the positive control particles of the present disclosure include a core covalently associated with a nucleic acid and an aminated polymer (e.g., as described herein). In some embodiments, the nucleic acid and/or the aminated polymer are covalently bound directly to a surface of the core. In some cases, the nucleic acid and/or the aminated polymer are indirectly covalently bound to a surface of the core, e.g., via a linker. In some embodiments, the linker may include one or more of the fluorescent particle binding molecules (e.g., aminated polymer molecules and/or nucleic acid molecules). For example, an aminated polymer molecule may be covalently bound to a nucleic acid molecule, wherein the nucleic acid molecule is covalently bound to the surface of the core either directly or through a linker.

The linkers employed may include, e.g., one or more reactive groups configured to stably associate two components of the positive control particle together. For example, suitable linkers, may include, but are not limited to, e.g., carboxyl to amine reactive groups, amine reactive groups, sulfhydryl reactive groups, aldehyde reactive groups, photoreactive groups, or hydroxyl reactive groups. In some embodiments, the linkers employ reactive linking chemistry such as where reactive linker pairs (e.g., as provided by a linker and, e.g., a chemical group of a core, an aminated polymer, or a nucleic acid as described herein) include, but are not limited to: maleimide/thiol; thiol/thiol; pyridyldithiol/thiol; succinimidyl iodoacetate/thiol; N-succinimidylester (NHS ester), sulfodicholorphenol ester (SDP ester), or pentafluorophenyl-ester (PFP ester)/amine; bissuccinimidylester/amine; imidoesters/amines; hydrazine or amine/aldehyde, dialdehyde or benzaldehyde; isocyanate/hydroxyl or amine; carbohydrate-periodate/hydrazine or amine; diazirine/aryl azide chemistry; pyridyldithiol/aryl azide chemistry; alkyne/azide; carboxy-carbodiimide/amine; amine/Sulfo-SMCC (Sulfosuccinimidyl 4-[N-maleimidomethyl]cyclohexane-1-carboxylate)/thiol and amine/BMPH (N-[β-Maleimidopropionic acid]hydrazide.TFA)/thiol; azide/triarylphosphine; nitrone/cyclooctyne; azide/tetrazine and formylbenzamide / hydrazino-nicotinamide. In certain embodiments, a linker employs a cycloaddition reaction, such as a [1+2]-cycloaddition, a [2+2]-cycloaddition, a [3+2]-cycloaddition, a [2+4]-cycloaddition, a [4+6]-cycloaddition, or cheleotropic reactions, including linkers that undergo a 1,3-dipolar cycloaddition (e.g., azide- alkyne Huisgen cycloaddition), a Diels-Alder reaction, an inverse electron demand Diels Alder cycloaddition, an ene reaction or a [2+2] photochemical cycloaddition reaction. In some embodiments, the linker may include an alkyl chain, an alkoxy chain, an alkenyl chain or an alkynyl chain, where the number of carbon atoms in the chain may vary, ranging in some instances from 2 to 25, such as 5 to 20, where one or more carbon atoms are replaced with, e.g., NH or CH₃-N as reactive functionalities for covalent bonding.

In some embodiments, the aminated polymer and the nucleic acid are layered on the core. In some embodiments, a plurality of aminated polymer layers of coating and/or nucleic acid layers of coating are covalently associated with the core. For example, the particle may include 5 or more aminated polymer layers and/or 5 or more nucleic acid layers. In some embodiments, the aminated polymer layers of coating alternate with the nucleic acid layers of coating. In other words, each layer includes either one or more of the aminated polymer molecules or one or more of the nucleic acid molecules and there are no two consecutive aminated polymer layers or nucleic acid layers. In these instances, the positive control particle may include multiple layers of each of the aminated polymer and the nucleic acid such as, e.g., 2 or more layers of each, or 3 or more, or 4 or more, or 5 or more, or 10 or more, etc. In other cases, the aminated polymer and the nucleic acid are evenly mixed and dispersed across the surface of the core. In some cases, the positive control particle includes a number of layers sufficient to bind an amount of an amine reactive dye and/or a DNA binding dye (e.g., of a given cell viability assay) such that the positive control particle is at least as bright as a cell stained using the same dye (e.g., during a cell viability assay). In these instances, the positive control particle may include a number of layers sufficient to bind an equal or greater amount of the amine reactive dye and/or the DNA binding dye as a non-viable cell of a specific cell viability assay (e.g., a non-viable mammalian tissue cell grown under specific conditions).

### Negative Control Particles

In certain embodiments, the control compositions of the present disclosure may include one or more negative control particles. By "negative control particle" is meant a particle, e.g., a bead, configured to have low affinity towards (e.g., have a high dissociation constant when interacting with) the amine binding fluorescent particle and/or the nucleic acid binding fluorescent particle a corresponding positive control particle (e.g., as described above) is configured to bind to relative to the positive control particle. In some embodiments, the negative control particle is configured to have substantially the same autofluorescence (i.e., the same inherent fluorescence) as the positive control particle. In some embodiments, the negative control particle is configured to have substantially the same autofluorescence as the cells of a given flow cytometry assay (e.g., cell viability assay). In these cases, the cells of interest may be mammalian cells. In some cases, the negative control particle is a bead.

In some instances, the negative control particle includes a core. In some cases, the core of the negative control particle may be substantially the same as the core of the positive control particle. For example, the core of the negative control particle may include the same material(s), be the same size and/or shape, have the same properties (e.g., autofluorescent properties), etc. as the core of the positive control particle. In some embodiments, the core of the negative control particle may essentially be the same as the core of the positive control particle. In some embodiments, the core of the negative control particle includes a material with low autofluorescence and/or low non-specific binding. In some embodiments, the core of the negative control particle is substantially non-autofluorescent and/or exhibits low non-specific binding, e.g., when considered as a whole.

In certain embodiments, the negative control particle does not include an aminated polymer or a nucleic acid (i.e., does not include any aminated polymer molecules or any nucleic acid molecules as described herein). In some cases, the negative control particle includes a polymer having substantially the same autofluorescence as the aminated polymer of the positive control (e.g., as described above), wherein the negative control polymer does not react or bind with amine reactive dyes. For example, the negative control particle may include a protein with substantially less lysine and arginine residues than the aminated polymer of the positive control particle. In some cases, the negative control particle includes a nucleic acid having substantially the same autofluorescence as the nucleic acid of the positive control (e.g., as described above), wherein the negative control nucleic acid does not react or bind with a DNA binding dye. For example, the negative control particle may include a nucleic acid with substantially no G-C base pairs, and the DNA binding dye may be 7-AAD. In other embodiments, the negative control nucleic acid is modified in such a way to prevent the binding of the DNA binding dye, or is a different type of nucleic acid than the positive control nucleic acid. For example, the negative control nucleic acid may be PNA and the positive control nucleic acid may be DNA.

As described above, in some embodiments, the negative control particle includes the core but does not include the polymer or the nucleic acid. In these instances, the core of the negative control particle may be conjugated with molecules in order to consume surface groups that may react with amine reactive dyes or DNA binding dyes. Such molecules may include, but are not limited to, e.g., polyethylene glycol (PEG) of various molecular weights and branching structures, zwitterionic polymers, poly(hydroxyfunctional acrylates), poly(2-oxazoline)s, poly(vinylpyrrolidone), poly(glycerol), peptides, proteins, and polysaccharides, or any combination thereof.. In some cases, a low non-specific binding polymer layer or coating such as, e.g., a silane layer or a layer of hydrophilic polymers (e.g., PEG, poly(vinyl pyrrolidone) (PVP), poly(acrylic acid) (PAA), poly(carboxybetaine acrylamide) (PCBAA) etc.), may be deposited on the surface of the core of the negative control particle.

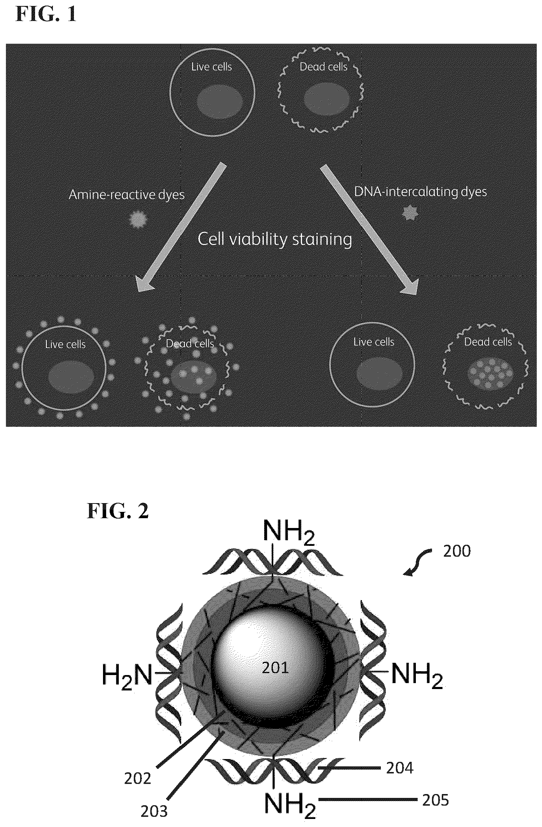

**FIG. 1** shows the mechanisms for two types of viability staining in accordance with an embodiment of the invention. At the top, two cells are depicted prior to being incubated with a cell viability dye. A viable cell (i.e., a live, healthy cell) having an intact cell membrane is shown on the left and a non-viable cell (i.e., a dead or dying cell) having a compromised cell membrane is shown on the right. Both viable and non-viable cells are then incubated with amine reactive dyes (middle left) and DNA-intercalating dyes (middle right) in order to differentiate between the viable and the non-viable cells. At the bottom, viable and non-viable cells are shown after incubation with either the amine reactive dyes or the DNA-intercalating dyes. For the amine reactive dye-based assay (bottom left), amine reactive dyes bind to amines on the proteins of the intact cell membrane of the viable cell, but fail to enter into the viable cell to bind with free amines in the cell interior. In contrast, for the non-viable cell amine reactive dyes bind with both amines of the compromised cell membrane and amines in the interior of a cell, resulting in a higher fluorescence intensity from the amine reactive dyes for the non-viable cell. For the DNA-intercalating dye-based assay (bottom right), DNA-intercalating dyes fail to enter into the viable cell to bind with dsDNA in the nucleus of the cell. In contrast, for the non-viable cell DNA-intercalating dyes penetrate all the way to the cell nucleus to bind directly within dsDNA molecules, resulting in a higher fluorescence intensity from the DNA-intercalating dyes for the non-viable cell. Accordingly, both the amine reactive dye-based assay and the DNA-intercalating dye-based assay are able to differentiate between viable and non-viable cells.

**FIG. 2** provides a representation of a positive CVC bead in accordance with an embodiment of the invention. The positive CVC bead **200** includes a core **201** consisting essentially of silica, a coating layer of DNA molecules **202** configured to bind to a DNA binding dye, and a coating layer of aminated polymer molecules **203** configured to bind to an amine-reactive dye. The DNA coating **202** includes a plurality of dsDNA sequences, each roughly 500 base pairs in length, spread relatively evenly throughout the surface of the core **201.** The DNA coating **202** is covalently attached to the surface of the core **201** either directly or via a linker, whereas the aminated polymer coating **203** is covalently associated with the core **201** through a covalent bond formed with the DNA coating **202.** The aminated polymer coating **203** includes a plurality of amine rich histone proteins spread relatively evenly throughout the DNA coating **202.**

A dsDNA molecule **204** used to bind a DNA binding dye and an amine group **205** used to bind an amine-reactive dye are depicted on the outside of the particle for illustrative purposes. Both the dsDNA molecules of the DNA coating **202** and the histone proteins of the aminated polymer coating **203** are positioned in a manner that allows dyes free flowing in a liquid solution to diffuse into sufficiently close proximity for the dsDNA molecules and the histone proteins (and amines thereof) to interact with the free-flowing dyes. This enables the amines of the aminated polymer coating **203** (e.g., **205)** to bind to the amine-reactive dyes of a liquid solution, and the dsDNA molecules of the DNA coating **202** (e.g., **204)** to bind to the DNA binding dyes of a liquid solution.

### METHODS OF MANUFACTURE

Aspects of the present disclosure also include methods of making (i.e., synthesizing or producing) the control particles as described herein. In certain embodiments, the methods of making may be used to produce positive control particles. In certain embodiments, the methods of making may be used to produce negative control particles. In some embodiments, one or more steps of the methods may be used to produce both positive control particles and negative control particles of the disclosure. In certain embodiments, particle synthesis methods include: obtaining a core; and covalently associating a polymer including an amine and a nucleic acid with the core to produce, e.g., a positive control particle.

In certain embodiments, the surface of the core includes a functional group such as, e.g., an amide, a maleimide, or a thiol group. In some embodiments, the method further includes surface functionalization of the core with the functional group. For example, the obtained core may include silica and the functionalization may include amination. In some embodiments, the method further includes modifying the functional group of the core to include a reactive functional group using, e.g., a reagent. For example, an aminated silica core may be modified to include a thiol group using a thiolating reagent such as, e.g., 2-iminothiolane. In some embodiments, the method further includes positively charging the core, e.g., when the core is covalently bound to a nucleic acid molecule (prior to being associated with an aminated polymer as described herein). In some embodiments, the method further includes negatively charging the core, e.g., when the core is covalently bound to an aminated polymer (prior to being associated with a nucleic acid as described herein).

In certain embodiments, the nucleic acid includes deoxyribonucleic acid (DNA) such as, e.g., double stranded DNA (dsDNA). In some embodiments, the particle synthesis method further includes modifying the DNA to include a functional group reactive to a functional group of the core and/or a functional group of the aminated polymer. For example, the core and/or the aminated polymer may be modified to include a thiol group and the DNA may be modified to include a maleimide group. In some embodiments, the particle synthesis method further includes modifying the DNA to include a functional group reactive to the reactive chemistry of a linker (i.e., wherein the linker is further configured to bind to the core and/or an aminated polymer). In some embodiments, the nucleic acid is covalently associated with the core by reacting the functional group of the DNA with the reactive functional group of the core and/or a linker associated with the core. In some embodiments, the nucleic acid is covalently associated with the core by reacting the functional group of the DNA with a functional group of the aminated polymer and/or a linker associated with the aminated polymer. In some embodiments, the 5' end and/or the 3' end of the DNA is functionalized. In some embodiments, the DNA is dsDNA and both strands of the dsDNA are functionalized. In some embodiments, the dsDNA is covalently bound to the core by reacting a functional group of a first strand of the dsDNA with a reactive functional group of the core (or linker thereof), and the dsDNA is covalently bound to the aminated polymer by reacting a functional group of a second strand of the dsDNA to a functional group of the aminated polymer (or linker thereof). In some embodiments, both strands of the dsDNA are covalently bound to aminated polymers.

In certain embodiments, the particle synthesis method further includes modifying the aminated polymer to include a functional group reactive to a functional group of the core and/or a functional group of the nucleic acid. For example, the core and/or the nucleic acid may be modified to include a maleimide group and the aminated polymer may be modified to include a thiol group. In some embodiments, the particle synthesis method further includes modifying the aminated polymer to include a functional group reactive to the reactive chemistry of a linker (i.e., wherein the linker is further configured to bind to the core and/or a nucleic acid). In some embodiments, the aminated polymer is covalently associated with the core by reacting the functional group of the aminated polymer with the reactive functional group of the core and/or a linker associated with the core. In some embodiments, the aminated polymer is covalently associated with the core by reacting the functional group of the aminated polymer with a functional group of the nucleic acid and/or a linker associated with the nucleic acid. In some embodiments, the aminated polymer is functionalized with two or more functional groups. For example, the aminated polymer may be a protein and two or more lysine residues of the protein may thiolated using, e.g., 2-iminothiolane. In some embodiments, the aminated polymer is covalently bound to the core by reacting a first functional group of the aminated polymer with the reactive functional group of the core (or linker thereof) and the aminated polymer is covalently bound to the nucleic acid by reacting a second functional group of the aminated polymer to a functional group of the nucleic acid (or linker thereof). In some embodiments, two or more functional groups of the aminated polymer are covalently bound to nucleic acids.

In certain embodiments, the aminated polymer and the nucleic acid are covalently associated with the core by alternatively layering coatings of the aminated polymer and the nucleic acid. For example, the nucleic acid may include dsDNA and may be modified to include a maleimide group on the 5' end of each strand of dsDNA, the aminated polymer may include a protein and may be modified to include two or more thiol groups, and the core may include a plurality of thiol groups (e.g., after modification). The layering may then include reacting the maleimide of a first 5' end of a first nucleic acid with a thiol group of the core, then reacting the maleimide of a second 5' end of the first nucleic acid with a first thiol group of a first aminated polymer, then reacting a second thiol group of the first aminated polymer with the maleimide of a first 5' end of a second nucleic acid, then reacting the maleimide of a second 5' end of the second nucleic acid with a first thiol group of a second aminated polymer, etc. In some embodiments, 5 or more rounds of layering are performed such that the control particle includes 5 or more nucleic acid layers and 5 or more aminated polymer layers. In some embodiments, each round of layering includes covalently associating a plurality of nucleic acids and a plurality of aminated polymers to the core.

In certain embodiments, the control particle is a positive control particle. In some embodiments, the particle synthesis method further includes incubating the positive control particle in a solution containing a plurality of amine reactive dyes and/or DNA binding dyes in order to create a labeled positive control particle. In certain embodiments, the control particle is a negative control particle. In some embodiments, the particle synthesis method further includes conjugating one or more polyethylene glycol (PEG) compounds (or other hydrophilic polymers as described above) to an amine of the negative control particle. In some embodiments, the negative control particle does not include the aminated polymer or the nucleic acid. In some embodiments, synthesizing the negative control particle includes conjugating one or more polyethylene glycol (PEG) compounds (or other hydrophilic polymers) to one or more amine groups of the core in order to prevent an amine reactive dye from reacting with the core.

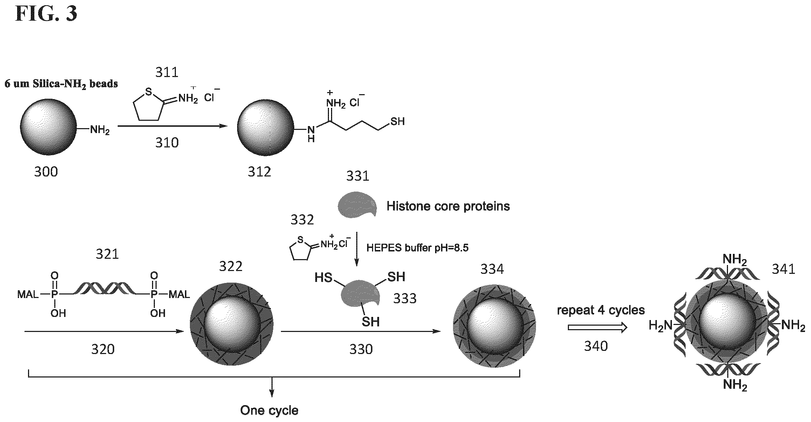

**FIG. 3** illustrates a method for synthesizing the positive control bead of **FIG. 2** in accordance with an embodiment of the invention. At step **300,** an aminated silica core is obtained, e.g., by generating a silica bead and performing surface functionalization in order to stably associate amines to the surface of the silica bead. At step **310,** the amines of the silica bead are modified by a thiolating reagent **311** (i.e., 2-iminothiolane) such that the bead includes thiol groups. The thiolated silica bead is then positively charged in order to obtain a thiolated, positively charged core **312.**

At step **320,** a DNA sequence, e.g., obtained from a salmon, is used to generate a dsDNA molecule. The dsDNA molecule is then modified to include a maleimide functional group reactive to the thiol group of the core at the 5' end or 3' end of each of the individual strands of the dsDNA molecule. A plurality of the maleimide functionalized dsDNA molecules **321** are then covalently bound to the thiolated, positively charged core in order to produce a silica core having a single coating layer of the dsDNA molecules **322.**

At step **330,** a histone protein **331** is obtained, e.g., by purifying the lysate of a mammalian cell. The histone protein is then modified by a thiolating reagent **332** (i.e., 2-iminothiolane) such that the protein includes thiol groups. A plurality of the thiolated histone proteins **333** are then covalently bound to the remaining maleimide group of a plurality of the dsDNA molecules bound to the silica core in order to produce a silica core having a single coating layer of the dsDNA molecules and a single layer of histone molecules **334.** At step **340,** step **320** is repeated in order to attach an additional dsDNA coating layer to a remaining thiol group of a plurality of the histone proteins of the outermost histone layer, and step **330** is repeated in order to attach an additional histone coating layer to the remaining maleimide group of a plurality of the dsDNA molecules of the outermost histone layer, until a positive control bead is generated having 5 aminated polymer (i.e., histone) coating layers [ and 5 nucleic acid (i.e., dsDNA) coating layers **341.**

### METHODS OF USE

Aspects of the present disclosure also include methods of using the subject control compositions. As described above, the control compositions may include a plurality of positive control particles, a plurality of negative control particles, or a plurality of both positive and negative control particles. As such, the methods of using the control compositions may include using the compositions to validate the functionality or verify the performance of particle analysis systems (e.g., flow cytometry systems), protocols, and reagents for use in flow cytometry assays. In some embodiments, the methods include using the control compositions to interpret flow cytometry data. In these instances, the methods may include using the control compositions to perform fluorescence compensation for flow cytometry data.

In some embodiments, the control compositions are used for fluorescence compensation by, e.g., correcting for fluorescence spillover by removing the signal of a given fluorescent particle (e.g., a given fluorophore or fluorochrome) from each secondary channel or detector. In other words, the control compositions are used to remove the signal of a given fluorescent particle from all the detectors or channels of a particle analyzer (e.g., flow cytometer) except the channel/detector specifically designated to measure the fluorescent particle. In some cases, fluorescence compensation may be performed for flow cytometric cell viability assays in order to, e.g., distinguish between live and dead cell populations in flow cytometry data. In some embodiments, flow cytometric cell viability assays are performed using an amine reactive dye to distinguish between live and dead cells. In certain embodiments, flow cytometric cell viability assays are performed using a DNA binding dye (e.g., DNA intercalating dye) to distinguish between live and dead cells.

In some embodiments, control compositions including positive control particles of the present disclosure are used to determine a compensation or spillover value for flow cytometric cell viability assays. In some cases, the range of wavelengths a given detection channel is configured to detect may be determined by the emission wavelength or wavelengths of the fluorophore to be detected, e.g., the fluorophore of an amine reactive dye or a DNA binding dye. To correct for fluorescence spillover, a positive control particle of the present disclosure may be used to determine the amount of fluorescence spillover produced by a given fluorescent particle, e.g., a given cell viability dye. In these instances, the positive control particles of the control composition may be contacted with one or more cell viability dyes (i.e., one or more amine reactive dyes and/or DNA binding dye) in order to label the positive control particles. The labeled control composition (i.e., including labeled positive control particles) may then be introduced into a flow cytometer in order to measure its fluorescence at one or more excitation wavelengths. A spillover or compensation value may then be calculated for each fluorescent particle (e.g., cell viability dye) of the cell viability assay using the fluorescence measurements generated from the labeled control composition. In some cases, the spillover or compensation values may be calculated by determining the amount of fluorescence detected in secondary channels (i.e., channels other than the detection channel specifically designated to measure a given fluorescent particle) for each fluorescent particle. The compensation/spillover value(s) may then be used to adjust flow cytometry data collected from the labeled sample cells of the cell viability assay. In some embodiments, the labeled control composition includes negative control particles, e.g., having substantially the same autofluorescence as the positive control particles and/or the cells of the give flow cytometry assay (e.g., cell viability assay). In these cases, fluorescence measurements generated from the negative control particles may be used to adjust or modify the fluorescence measurements generated from the positive control particles prior to calculating the compensation/spillover value(s).

In certain embodiments, the subject control compositions (e.g., the negative and positive control particles of the invention) may be used to perform fluorescence minus one (FMO) controls. For example, the flow cytometry assay may include three or more fluorescent particles: one or more cell viability dyes (i.e., amine reactive dyes or DNA binding dyes) and one or more other fluorescent particles (e.g., antibody-fluorophore conjugates, nucleic acid probes, etc.). In these cases, a population of positive control particles may be generated for each fluorescent particle, such that the control composition includes positive control particles configured to bind every fluorescent particle of the assay except one for each fluorescent particle. The control composition may then be labeled (i.e., by incubation with all the dyes of the assay), and the labeled control composition may then be introduced into a flow cytometer in order to measure its fluorescence at one or more excitation wavelengths. A spillover or compensation value may then be calculated for each fluorescent particle (e.g., cell viability dye) of the cell viability assay using the fluorescence measurements generated from the labeled control composition.

### Sample Analysis

As discussed above, the subject control compositions may be used in the analysis of a cellular sample such as, e.g., in determining the viable and non-viable cells of the sample by performing a flow cytometric cell viability assay. Cells that may be present in the sample include eukaryotic cells (e.g., mammalian cells) and/or prokaryotic cells (e.g., bacterial cells or archaeal cells). Samples may be obtained from an *in vitro* source (e.g., a suspension of cells from laboratory cells grown in culture) or from an *in vivo* source (e.g., a mammalian subject, a human subject, etc.). In some embodiments, the cellular sample is obtained from an *in vitro* source. *In vitro* sources include, but are not limited to, prokaryotic (e.g., bacterial, archaeal) cell cultures, environmental samples that contain prokaryotic and/or eukaryotic (e.g., mammalian, protest, fungal, etc.) cells, eukaryotic cell cultures (e.g., cultures of established cell lines, cultures of known or purchased cell lines, cultures of immortalized cell lines, cultures of primary cells, cultures of laboratory yeast, etc.), tissue cultures, and the like. *In vitro* source may further include organismic tissue, including both healthy and diseased tissue (e.g., cancerous, malignant, necrotic, etc.), such as, e.g., sections of the skin, respiratory system, gastrointestinal system, cardiovascular system, genitourinary tracts, tumors, organs, etc. In other instances, cells of a cellular sample may be obtained from a biologically produced fluid such as, e.g., blood, mucus, lymphatic fluid, synovial fluid, cerebrospinal fluid, saliva, bronchoalveolar lavage, amniotic fluid, amniotic cord blood, urine, vaginal fluid and/or semen.

In certain embodiments the source of the sample is a "mammal" or "mammalian", where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In some instances, the subjects are humans. The methods may be applied to samples obtained from human subjects of both genders and at any stage of development (i.e., neonates, infant, juvenile, adolescent, adult), where in certain embodiments the human subject is a juvenile, adolescent or adult. While the present invention may be applied to samples from a human subject, it is to be understood that the methods may also be carried-out on samples from other animal subjects (that is, in "non-human subjects") such as, but not limited to, birds, mice, rats, dogs, cats, livestock and horses.

In certain embodiments, control compositions employed in the present methods may be employed in a flow cytometric protocol (e.g., to analyze a sample, such as those discussed above). In practicing such methods, a sample, and a control composition (e.g., in a flow stream of a flow cytometer) are irradiated with light from a light source. In some embodiments, the light source is a broadband light source, emitting light having a broad range of wavelengths, such as for example, spanning 50 nm or more, such as 100 nm or more, such as 150 nm or more, such as 200 nm or more, such as 250 nm or more, such as 300 nm or more, such as 350 nm or more, such as 400 nm or more and including spanning 500 nm or more. For example, one suitable broadband light source emits light having wavelengths from 200 nm to 1500 nm. Another example of a suitable broadband light source includes a light source that emits light having wavelengths from 400 nm to 1000 nm. Where methods include irradiating with a broadband light source, broadband light source protocols of interest may include, but are not limited to, a halogen lamp, deuterium arc lamp, xenon arc lamp, stabilized fiber-coupled broadband light source, a broadband LED with continuous spectrum, superluminescent emitting diode, semiconductor light emitting diode, wide spectrum LED white light source, an multi-LED integrated white light source, among other broadband light sources or any combination thereof.

In other embodiments, methods of embodiments of the invention include irradiating with a narrow band light source emitting a particular wavelength or a narrow range of wavelengths, such as for example with a light source which emits light in a narrow range of wavelengths like a range of 50 nm or less, such as 40 nm or less, such as 30 nm or less, such as 25 nm or less, such as 20 nm or less, such as 15 nm or less, such as 10 nm or less, such as 5 nm or less, such as 2 nm or less and including light sources which emit a specific wavelength of light (i.e., monochromatic light). Where methods include irradiating with a narrow band light source, narrow band light source protocols of interest may include, but are not limited to, a narrow wavelength LED, laser diode or a broadband light source coupled to one or more optical bandpass filters, diffraction gratings, monochromators or any combination thereof.

In certain embodiments, methods include irradiating the sample and control composition with one or more lasers. As discussed above, the type and number of lasers will vary depending on the sample and the control composition as well as desired light collected and may be a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the methods include irradiating the flow stream with a dye laser, such as a stilbene, coumarin or rhodamine laser. In yet other instances, methods include irradiating the flow stream with a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, methods include irradiating the flow stream with a solid-state laser, such as a ruby laser, an Nd:YAG laser, NdCrYAG laser, Er:YAG laser, Nd:YLF laser, Nd:YVO₄ laser, Nd:YCa₄O(BO₃)₃ laser, Nd:YCOB laser, titanium sapphire laser, thulim YAG laser, ytterbium YAG laser, ytterbium₂O₃ laser or cerium doped lasers and combinations thereof.

The sample and the control composition may be irradiated with one or more of the above mentioned light sources, such as 2 or more light sources, such as 3 or more light sources, such as 4 or more light sources, such as 5 or more light sources and including 10 or more light sources. The light source may include any combination of types of light sources. For example, in some embodiments, the methods include irradiating the sample and the control composition in the flow stream with an array of lasers, such as an array having one or more gas lasers, one or more dye lasers and one or more solid-state lasers.

The sample and the control composition may be irradiated with wavelengths ranging from 200 nm to 1500 nm, such as from 250 nm to 1250 nm, such as from 300 nm to 1000 nm, such as from 350 nm to 900 nm and including from 400 nm to 800 nm. For example, where the light source is a broadband light source, the sample and the control composition may be irradiated with wavelengths from 200 nm to 900 nm. In other instances, where the light source includes a plurality of narrow band light sources, the sample and control composition may be irradiated with specific wavelengths in the range from 200 nm to 900 nm. For example, the light source may be plurality of narrow band LEDs (1 nm - 25 nm) each independently emitting light having a range of wavelengths between 200 nm to 900 nm. In other embodiments, the narrow band light source includes one or more lasers (such as a laser array) and the sample and control composition are irradiated with specific wavelengths ranging from 200 nm to 700 nm, such as with a laser array having gas lasers, excimer lasers, dye lasers, metal vapor lasers and solid-state laser as described above.

Where more than one light source is employed, the sample and the control composition may be irradiated with the light sources simultaneously or sequentially, or a combination thereof. For example, the sample and control composition may be simultaneously irradiated with each of the light sources. In other embodiments, the flow stream is sequentially irradiated with each of the light sources. Where more than one light source is employed to irradiate the sample and the control composition sequentially, the time each light source irradiates the sample and control composition may independently be 0.001 microseconds or more, such as 0.01 microseconds or more, such as 0.1 microseconds or more, such as 1 microsecond or more, such as 5 microseconds or more, such as 10 microseconds or more, such as 30 microseconds or more and including 60 microseconds or more. For example, methods may include irradiating the sample and control composition with the light source (e.g., laser) for a duration which ranges from 0.001 microseconds to 100 microseconds, such as from 0.01 microseconds to 75 microseconds, such as from 0.1 microseconds to 50 microseconds, such as from 1 microsecond to 25 microseconds and including from 5 microseconds to 10 microseconds. In embodiments where the sample and control composition are sequentially irradiated with two or more light sources, the duration the sample and control composition are irradiated by each light source may be the same or different.

The time period between irradiation by each light source may also vary, as desired, being separated independently by a delay of 0.001 microseconds or more, such as 0.01 microseconds or more, such as 0.1 microseconds or more, such as 1 microsecond or more, such as 5 microseconds or more, such as by 10 microseconds or more, such as by 15 microseconds or more, such as by 30 microseconds or more and including by 60 microseconds or more. For example, the time period between irradiation by each light source may range from 0.001 microseconds to 60 microseconds, such as from 0.01 microseconds to 50 microseconds, such as from 0.1 microseconds to 35 microseconds, such as from 1 microsecond to 25 microseconds and including from 5 microseconds to 10 microseconds. In certain embodiments, the time period between irradiation by each light source is 10 microseconds. In embodiments where the sample and the control composition are sequentially irradiated by more than two (i.e., 3 or more) light sources, the delay between irradiation by each light source may be the same or different.

The sample and the and control composition may be irradiated continuously or in discrete intervals. In some instances, methods include irradiating the sample and the control composition with the light source continuously. In other instances, the sample and control composition are irradiated with the light source in discrete intervals, such as irradiating every 0.001 millisecond, every 0.01 millisecond, every 0.1 millisecond, every 1 millisecond, every 10 milliseconds, every 100 milliseconds and including every 1000 milliseconds, or some other interval.

Depending on the light source, the sample and control composition may be irradiated from a distance which varies such as 0.01 mm or more, such as 0.05 mm or more, such as 0.1 mm or more, such as 0.5 mm or more, such as 1 mm or more, such as 2.5 mm or more, such as 5 mm or more, such as 10 mm or more, such as 15 mm or more, such as 25 mm or more and including 50 mm or more. Also, the angle or irradiation may also vary, ranging from 10° to 90°, such as from 15° to 85°, such as from 20° to 80°, such as from 25° to 75° and including from 30° to 60°, for example at a 90° angle.

In certain instances, the sample and the and control composition may be irradiated with a plurality of angularly deflected beams of frequency-shifted light and a cell in the flow stream is imaged by fluorescence imaging using radiofrequency tagged emission (FIRE) to generate a frequency-encoded image, such as those described in Diebold, et al. Nature Photonics Vol. 7(10); 806-810 (2013) as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894.

In embodiments, light from the irradiated sample and the irradiated control composition is conveyed to a light detection system and measured by one or more photodetectors. In practicing the subject methods, light from the sample and control composition is conveyed to three or more wavelength separators that are each configured to pass light having a predetermined spectral range. The spectral ranges of light from each of the wavelength separators are conveyed to one or more light detection modules having optical components that are configured to convey light having a predetermined sub-spectral range to the photodetectors.

Light may be measured with the light detection systems continuously or in discrete intervals. In some instances, methods include taking measurements of the light continuously. In other instances, the light is measured in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

Measurements of the collected light may be taken one or more times during the subject methods, such as 2 or more times, such as 3 or more times, such as 5 or more times and including 10 or more times. In certain embodiments, the light propagation is measured 2 or more times, with the data in certain instances being averaged.

In some embodiments, methods include adjusting the light before detecting the light with the subject light detection systems. For example, the light from the sample and the control composition may be passed through one or more lenses, mirrors, pinholes, slits, gratings, light refractors, and any combination thereof. In some instances, the collected light is passed through one or more focusing lenses, such as to reduce the profile of the light directed to the light detection system or optical collection system as described above. In other instances, the emitted light from the sample and the emitted light from the control composition is passed through one or more collimators to reduce light beam divergence conveyed to the light detection system.

### SYSTEMS

Aspects of the invention additionally include systems configured to perform the above-described methods. Systems of interest include a processor configured to display results in accordance with embodiments of the invention. In embodiments, the subject processors are operated in conjunction with programmable logic that may be implemented in hardware, software, firmware, or any combination thereof in order to display results. In some such embodiments, the system includes a display configured to portray the visualization. Any suitable display may be employed. The subject display may include, but is not limited to, a monitor, a tablet computer, a smartphone, or other electronic device configured to present graphical interfaces. In this section, the word "sample" may refer to a cellular sample and a control composition of the present disclosure, or just the cellular sample depending on context.

The subject programmable logic may be implemented in any of a variety of devices such as specifically programmed event processing computers, wireless communication devices, integrated circuit devices, or the like. In some embodiments, the programable logic may be executed by a specifically programmed processor, which may include one or more processors, such as one or more digital signal processors (DSPs), configurable microprocessors, application specific integrated circuits (ASICs), field programmable gate arrays (FPGAs), or other equivalent integrated or discrete logic circuitry. A combination of computing devices, e.g., a combination of a DSP and a microprocessor, a plurality of microprocessors, one or more microprocessors in conjunction with a DSP core, or any other such configuration in at least partial data connectivity may implement one or more of the features described herein.

In certain instances, the system is or includes a particle analyzer. Particle analyzers of interest may include a flow cell for transporting particles in a flow stream, a light source for irradiating the particles in the flow stream at an interrogation point, and a particle-modulated light detector for detecting particle-modulated light. In certain embodiments, the particle analyzer is a flow cytometer. In some cases where the particle analyzer is a flow cytometer, said flow cytometer is a full spectrum flow cytometer.

As discussed herein, a "flow cell" is described in its conventional sense to refer to a component, such as a cuvette, containing a flow channel having a liquid flow stream for transporting particles in a sheath fluid. Cuvettes of interest include containers having a passage running therethrough. The flow stream may include a liquid sample injected from a sample tube. Flow cells of interest include a light-accessible flow channel. In some instances, the flow cell includes transparent material (e.g., quartz) that permits the passage of light therethrough. In some embodiments, the flow cell is a stream-in-air flow cell in which light interrogation of the particles occurs outside of the flow cell (i.e., in free space).

In some cases, the flow stream is configured for irradiation with light from a light source at an interrogation point. The flow stream for which the flow channel is configured may include a liquid sample injected from a sample tube. In certain embodiments, the flow stream may include a narrow, rapidly flowing stream of liquid that is arranged such that linearly segregated particles transported therein are separated from each other in a single-file manner. The "interrogation point" discussed herein refers to a region within the flow cell in which the particle is irradiated by light from the light source, e.g., for analysis. The size of the interrogation point may vary as desired. For example, where 0 µm represents the axis of light emitted by the light source, the interrogation point may range from - 100 µm to 100 µm, such as -50 µm to 50 µm, such as -25 µm to 40 µm, and including -15 µm to 30 µm.

After particles are irradiated in the flow cell, particle-modulated light may be observed. By "particle-modulated light" it is meant light that is received from the particles in the flow stream following the irradiation of the particles with light from the light source. In some cases, the particle-modulated light is side-scattered light. As discussed herein, side-scattered light refers to light refracted and reflected from the surfaces and internal structures of the particle. In additional embodiments, the particle-modulated light includes forward-scattered light (i.e., light that travels through or around the particle in mostly a forward direction). In still other cases, the particle-modulated light includes fluorescent light (i.e., light emitted from a fluorochrome following irradiation with excitation wavelength light).

As discussed above, aspects of the invention also include a light source configured to irradiate particles passing through the flow cell at an interrogation point. Any convenient light source may be employed as the light source described herein. In some embodiments, the light source is a laser. In embodiments, the laser may be any convenient laser, such as a continuous wave laser. For example, the laser may be a diode laser, such as an ultraviolet diode laser, a visible diode laser and a near-infrared diode laser. In other embodiments, the laser may be a helium-neon (HeNe) laser. In some instances, the laser is a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the subject flow cytometers include a dye laser, such as a stilbene, coumarin or rhodamine laser. In yet other instances, lasers of interest include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. In still other instances, the subject flow cytometers include a solid-state laser, such as a ruby laser, an Nd:YAG laser, NdCrYAG laser, Er:YAG laser, Nd:YLF laser, Nd:YVO₄ laser, Nd:YCa₄O(BO₃)₃ laser, Nd:YCOB laser, titanium sapphire laser, thulim YAG laser, ytterbium YAG laser, ytterbium₂O₃ laser or cerium doped lasers and combinations thereof.

Laser light sources according to certain embodiments may also include one or more optical adjustment components. In certain embodiments, the optical adjustment component is located between the light source and the flow cell, and may include any device that is capable of changing the spatial width of irradiation or some other characteristic of irradiation from the light source, such as for example, irradiation direction, wavelength, beam width, beam intensity and focal spot. Optical adjustment protocols may include any convenient device which adjusts one or more characteristics of the light source, including but not limited to lenses, mirrors, filters, fiber optics, wavelength separators, pinholes, slits, collimating protocols and combinations thereof. In certain embodiments, flow cytometers of interest include one or more focusing lenses. The focusing lens, in one example, may be a de-magnifying lens. In still other embodiments, flow cytometers of interest include fiber optics.

Where the optical adjustment component is configured to move, the optical adjustment component may be configured to be moved continuously or in discrete intervals, such as for example in 0.01 µm or greater increments, such as 0.05 µm or greater, such as 0.1 µm or greater, such as 0.5 µm or greater such as 1 µm or greater, such as 10 µm or greater, such as 100 µm or greater, such as 500 µm or greater, such as 1 mm or greater, such as 5 mm or greater, such as 10 mm or greater and including 25 mm or greater increments.

Any displacement protocol may be employed to move the optical adjustment component structures, such as coupled to a moveable support stage or directly with a motor actuated translation stage, leadscrew translation assembly, geared translation device, such as those employing a stepper motor, servo motor, brushless electric motor, brushed DC motor, micro-step drive motor, high resolution stepper motor, among other types of motors.

The light source may be positioned any suitable distance from the flow cell, such as where the light source and the flow cell are separated by 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source may be positioned at any suitable angle relative to the flow cell, such as at an angle ranging from 10 degrees to 90 degrees, such as from 15 degrees to 85 degrees, such as from 20 degrees to 80 degrees, such as from 25 degrees to 75 degrees and including from 30 degrees to 60 degrees, for example at a 90 degree angle.

In some embodiments, light sources of interest include a plurality of lasers configured to provide laser light for discrete irradiation of the flow stream, such as 2 lasers or more, such as 3 lasers or more, such as 4 lasers or more, such as 5 lasers or more, such as 10 lasers or more, and including 15 lasers or more configured to provide laser light for discrete irradiation of the flow stream. Depending on the desired wavelengths of light for irradiating the flow stream, each laser may have a specific wavelength that varies from 200 nm to 1500 nm, such as from 250 nm to 1250 nm, such as from 300 nm to 1000 nm, such as from 350 nm to 900 nm and including from 400 nm to 800 nm. In certain embodiments, lasers of interest may include one or more of a 405 nm laser, a 488 nm laser, a 561 nm laser and a 635 nm laser.

As discussed above, particle analyzers of interest may further include one or more particle-modulated light detectors for detecting particle-modulated light intensity data. In some embodiments, the particle-modulated light detector(s) include one or more forward-scattered light detectors configured to detect forward-scattered light. For example, the subject particle analyzers may include 1 forward-scattered light detector or multiple forward-scattered light detectors, such as 2 or more, such as 3 or more, such as 4 or more, and including 5 or more. In certain embodiments, particle analyzers include 1 forward-scattered light detector. In other embodiments, particle analyzers include 2 forward-scattered light detectors.

Any convenient detector for detecting collected light may be used in the forward-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from 0.1 cm² to 8 cm², such as from 0.5 cm² to 7 cm² and including from 1 cm² to 5 cm².

In embodiments, the forward-scattered light detector is configured to measure light continuously or in discrete intervals. In some instances, detectors of interest are configured to take measurements of the collected light continuously. In other instances, detectors of interest are configured to take measurements in discrete intervals, such as measuring light every 0.001 millisecond, every 0.01 millisecond, every 0.1 millisecond, every 1 millisecond, every 10 milliseconds, every 100 milliseconds and including every 1000 milliseconds, or some other interval.

In additional embodiments, the one or more particle-modulated light detector(s) may include one or more side-scattered light detectors for detecting side-scatter wavelengths of light (i.e., light refracted and reflected from the surfaces and internal structures of the particle). In some embodiments, particle analyzers include a single side-scattered light detector. In other embodiments, particle analyzers include multiple side-scattered light detectors, such as 2 or more, such as 3 or more, such as 4 or more, and including 5 or more.

Any convenient detector for detecting collected light may be used in the side-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from 0.1 cm² to 8 cm², such as from 0.5 cm² to 7 cm² and including from 1 cm² to 5 cm².

In embodiments, the subject particle analyzers also include a fluorescent light detector configured to detect one or more fluorescent wavelengths of light. In other embodiments, particle analyzers include multiple fluorescent light detectors such as 2 or more, such as 3 or more, such as 4 or more, 5 or more, 10 or more, 15 or more, and including 20 or more.

Any convenient detector for detecting collected light may be used in the fluorescent light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. In certain embodiments, the collected light is measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. In certain embodiments, the detector is a photomultiplier tube, such as a photomultiplier tube having an active detecting surface area of each region that ranges from 0.01 cm² to 10 cm², such as from 0.05 cm² to 9 cm², such as from, such as from 0.1 cm² to 8 cm², such as from 0.5 cm² to 7 cm² and including from 1 cm² to 5 cm².

Where the subject particle analyzers include multiple fluorescent light detectors, each fluorescent light detector may be the same, or the collection of fluorescent light detectors may be a combination of different types of detectors. For example, where the subject particle analyzers include two fluorescent light detectors, in some embodiments the first fluorescent light detector is a CCD-type device and the second fluorescent light detector (or imaging sensor) is a CMOS-type device. In other embodiments, both the first and second fluorescent light detectors are CCD-type devices. In yet other embodiments, both the first and second fluorescent light detectors are CMOS-type devices. In still other embodiments, the first fluorescent light detector is a CCD-type device and the second fluorescent light detector is a photomultiplier tube (PMT). In still other embodiments, the first fluorescent light detector is a CMOS-type device and the second fluorescent light detector is a photomultiplier tube. In yet other embodiments, both the first and second fluorescent light detectors are photomultiplier tubes.

In embodiments of the present disclosure, fluorescent light detectors of interest are configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths. In some embodiments, 2 or more detectors in the particle analyzers as described herein are configured to measure the same or overlapping wavelengths of collected light.

In some embodiments, fluorescent light detectors of interest are configured to measure collected light over a range of wavelengths (e.g., 200 nm - 1000 nm). In certain embodiments, detectors of interest are configured to collect spectra of light over a range of wavelengths. For example, particle analyzers may include one or more detectors configured to collect spectra of light over one or more of the wavelength ranges of 200 nm - 1000 nm. In yet other embodiments, detectors of interest are configured to measure light emitted by a sample in the flow stream at one or more specific wavelengths. For example, particle analyzers may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof. In certain embodiments, one or more detectors may be configured to be paired with specific fluorophores, such as those used with the sample in a fluorescence assay.

In some embodiments, particle analyzers include one or more wavelength separators positioned between the flow cell and the particle-modulated light detector(s). The term "wavelength separator" is used herein in its conventional sense to refer to an optical component that is configured to separate light collected from the sample into predetermined spectral ranges. In some embodiments, particle analyzers include a single wavelength separator. In other embodiments, particle analyzers include a plurality of wavelength separators, such as 2 or more wavelength separators, such as 3 or more, such as 4 or more, such as 5 or more, such as 6 or more, such as 7 or more, such as 8 or more, such as 9 or more, such as 10 or more, such as 15 or more, such as 25 or more, such as 50 or more, such as 75 or more and including 100 or more wavelength separators. In some embodiments, the wavelength separator is configured to separate light collected from the sample into predetermined spectral ranges by passing light having a predetermined spectral range and reflecting one or more remaining spectral ranges of light. In other embodiments, the wavelength separator is configured to separate light collected from the sample into predetermined spectral ranges by passing light having a predetermined spectral range and absorbing one or more remaining spectral ranges of light. In yet other embodiments, the wavelength separator is configured to spatially diffract light collected from the sample into predetermined spectral ranges. Each wavelength separator may be any convenient light separation protocol, such as one or more dichroic mirrors, bandpass filters, diffraction gratings, beam splitters or prisms. In some embodiments, the wavelength separator is a prism. In other embodiments, the wavelength separator is a diffraction grating. In certain embodiments, wavelength separators in the subject light detection systems are dichroic mirrors.

Suitable flow cytometry systems may include, but are not limited to those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1):17-28; Linden, et. al., Semin Throm Hemost. 2004 Oct;30(5):502-11; Alison, et al. J Pathol, 2010 Dec; 222(4):335-344; and Herbig, et al. (2007) Crit Rev Ther Drug Carrier Syst. 24(3):203-255; In certain instances, flow cytometry systems of interest include BD Biosciences FACSCanto^{™} flow cytometer, BD Biosciences FACSCanto^{™} II flow cytometer, BD Accuri^{™} flow cytometer, BD Accuri^{™} C6 Plus flow cytometer, BD Biosciences FAeSeelesta^{™} flow cytometer, BD Biosciences FACSLyric^{™} flow cytometer, BD Biosciences FACSVerse^{™} flow cytometer, BD Biosciences FACSymphony^{™} flow cytometer, BD Biosciences LSRFortessa^{™} flow cytometer, BD Biosciences LSRFortessa^{™} X-20 flow cytometer, BD Biosciences FACSPresto^{™} flow cytometer, BD Biosciences FAeSVia^{™} flow cytometer and BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences Aria^{™} cell sorter, BD Biosciences FACSAria^{™} II cell sorter, BD Biosciences FACSAria^{™} III cell sorter, BD Biosciences FACSAria^{™} Fusion cell sorter and BD Biosciences FAeSMelody^{™} cell sorter, BD Biosciences FACSymphony^{™} S6BD, FACSDiscover^{™} S8 Cell Sorter cell sorter or the like.

In some embodiments, the subject systems are flow cytometric systems, such those described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766;

In certain instances, flow cytometry systems of the invention are configured for imaging particles in a flow stream by fluorescence imaging using radiofrequency tagged emission (FIRE), such as those described in Diebold, et al. Nature Photonics Vol. 7(10); 806-810 (2013) as well as described in U.S. Patent Nos. 9,423,353; 9,784,661; 9,983,132; 10,006,852; 10,078,045; 10,036,699; 10,222,316; 10,288,546; 10,324,019; 10,408,758; 10,451,538; 10,620,111; and U.S. Patent Publication Nos. 2017/0133857; 2017/0328826; 2017/0350803; 2018/0275042; 2019/0376895 and 2019/0376894.. In such instances, flow cytometric data may include image data of particles, e.g., cells present in the sample. See, e.g., Schraivogel et al., Science Vol. 375(6578); 315-320 (2022)., , as well as U.S. Provisional Patent Application Serial No. 63/256,974,).. An example of such a system is FACSDiscover^{™} S8 Cell Sorter cell sorter.

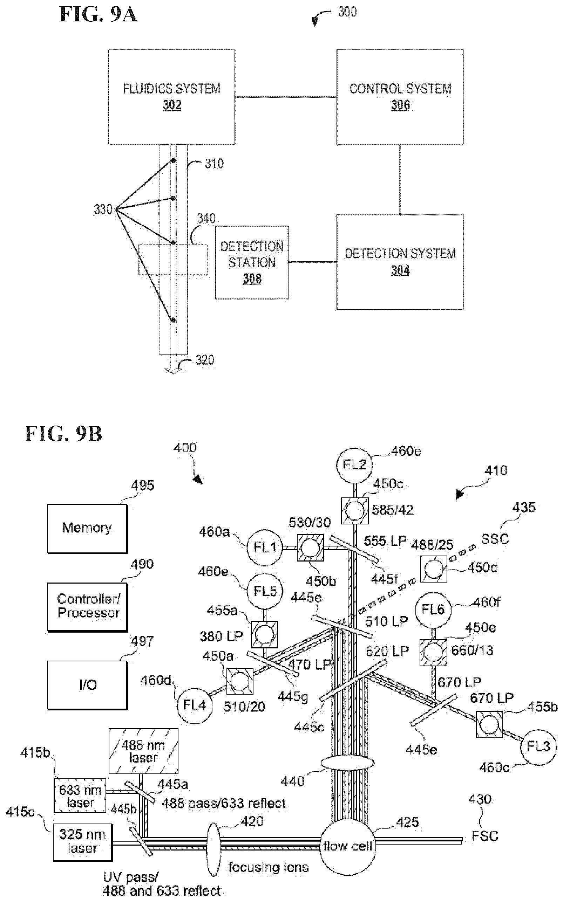

In some embodiments, systems are particle analyzers where the particle analysis system **300 (****FIG. 9A****)** can be used to analyze and characterize particles, with or without physically sorting the particles into collection vessels. **FIG. 9A** shows a functional block diagram of a particle analysis system for computational based sample analysis and particle characterization. In some embodiments, the particle analysis system **300** is a flow system. The particle analysis system **300** shown in **FIG. 9A** can be configured to perform, in whole or in part, the methods described herein such as. The particle analysis system **300** includes a fluidics system **302.** The fluidics system **302** can include or be coupled with a sample tube **310** and a moving fluid column within the sample tube in which particles **330** (e.g., cells) of a sample move along a common sample path **320.**

The particle analysis system **300** includes a detection system **304** configured to collect a signal from each particle as it passes one or more detection stations along the common sample path. A detection station **308** generally refers to a monitored area **340** of the common sample path. Detection can, in some implementations, include detecting light or one or more other properties of the particles **330** as they pass through the monitored area **340.** In **FIG. 9A****,** one detection station **308** with one monitored area **340** is shown. Some implementations of the particle analysis system **300** can include multiple detection stations. Furthermore, some detection stations can monitor more than one area.

Each signal is assigned a signal value to form a data point for each particle. As described above, this data can be referred to as event data. The data point can be a multidimensional data point including values for respective properties measured for a particle. The detection system **304** is configured to collect a succession of such data points in a first-time interval.

The particle analysis system **300** can also include a control system **306.** The control system **306** can include one or more processors, an amplitude control circuit and/or a frequency control circuit. The control system shown can be operationally associated with the fluidics system **302.** The control system can be configured to generate a calculated signal frequency for at least a portion of the first-time interval based on a Poisson distribution and the number of data points collected by the detection system **304** during the first time interval. The control system **306** can be further configured to generate an experimental signal frequency based on the number of data points in the portion of the first time interval. The control system **306** can additionally compare the experimental signal frequency with that of a calculated signal frequency or a predetermined signal frequency.

**FIG. 9B** shows a system **400** for flow cytometry in accordance with an illustrative embodiment of the present invention. The system **400** includes a flow cytometer **410,** a controller/processor **490** and a memory **495.** The flow cytometer **410** includes one or more excitation lasers **415a-415c,** a focusing lens **420,** a flow chamber **425,** a forward scatter detector **430,** a side scatter detector **435,** a fluorescence collection lens **440,** one or more beam splitters **445a-445g,** one or more bandpass filters **450a-450e,** one or more longpass ("LP") filters **455a-455b,** and one or more fluorescent detectors **460a-460f.**

The excitation lasers **415a-415c** emit light in the form of a laser beam. The wavelengths of the laser beams emitted from excitation lasers **415a-415c** are 488 nm, 633 nm, and 325 nm, respectively, in the example system of **FIG. 9B****.** The laser beams are first directed through one or more of beam splitters **445a** and **945b.** Beam splitter **445a** transmits light at 488 nm and reflects light at 633 nm. Beam splitter **445b** transmits UV light (light with a wavelength in the range of 10 to 400 nm) and reflects light at 488 nm and 633 nm.

The laser beams are then directed to a focusing lens **420,** which focuses the beams onto the portion of a fluid stream where particles of a sample are located, within the flow chamber **425.** The flow chamber is part of a fluidics system which directs particles, typically one at a time, in a stream to the focused laser beam for interrogation. The flow chamber can comprise a flow cell in a benchtop cytometer or a nozzle tip in a stream-in-air cytometer.

The light from the laser beam(s) interacts with the particles in the sample by diffraction, refraction, reflection, scattering, and absorption with re-emission at various different wavelengths depending on the characteristics of the particle such as its size, internal structure, and the presence of one or more fluorescent molecules attached to or naturally present on or in the particle. The fluorescence emissions as well as the diffracted light, refracted light, reflected light, and scattered light may be routed to one or more of the forward scatter detector **430,** the side scatter detector **435,** and the one or more fluorescent detectors **460a-460f** through one or more of the beam splitters **445a-445g,** the bandpass filters **450a-450e,** the longpass filters **455a-955b,** and the fluorescence collection lens **440.** The fluorescence collection lens **440** collects light emitted from the particle- laser beam interaction and routes that light towards one or more beam splitters and filters. Bandpass filters, such as bandpass filters **450a-450e,** allow a narrow range of wavelengths to pass through the filter. For example, bandpass filter **450a** is a 510/20 filter. The first number represents the center of a spectral band. The second number provides a range of the spectral band. Thus, a 510/20 filter extends 10 nm on each side of the center of the spectral band, or from 500 nm to 520 nm. Shortpass filters transmit wavelengths of light equal to or shorter than a specified wavelength. Longpass filters, such as longpass filters **455a-455b,** transmit wavelengths of light equal to or longer than a specified wavelength of light. For example, longpass filter **455a,** which is a 670 nm longpass filter, transmits light equal to or longer than 670 nm. Filters are often selected to optimize the specificity of a detector for a particular fluorescent dye. The filters can be configured so that the spectral band of light transmitted to the detector is close to the emission peak of a fluorescent dye.

Beam splitters direct light of different wavelengths in different directions. Beam splitters can be characterized by filter properties such as shortpass and longpass. For example, beam splitter **445g** is a 620 SP beam splitter, meaning that the beam splitter **445g** transmits wavelengths of light that are 620 nm or shorter and reflects wavelengths of light that are longer than 620 nm in a different direction. In one embodiment, the beam splitters **445a-445g** can comprise optical mirrors, such as dichroic mirrors.

The forward scatter detector **430** is positioned slightly off axis from the direct beam through the flow cell and is configured to detect diffracted light, the excitation light that travels through or around the particle in mostly a forward direction. The intensity of the light detected by the forward scatter detector is dependent on the overall size of the particle. The forward scatter detector can include a photodiode. The side scatter detector **435** is configured to detect refracted and reflected light from the surfaces and internal structures of the particle, and tends to increase with increasing particle complexity of structure. The fluorescence emissions from fluorescent molecules associated with the particle can be detected by the one or more fluorescent detectors **460a-460f.** The side scatter detector **435** and fluorescent detectors can include photomultiplier tubes. The signals detected at the forward scatter detector **430,** the side scatter detector **435** and the fluorescent detectors can be converted to electronic signals (voltages) by the detectors. This data can provide information about the sample.

One of skill in the art will recognize that a flow cytometer in accordance with an embodiment of the present invention is not limited to the flow cytometer depicted in **FIG. 9B****,** but can include any flow cytometer known in the art. For example, a flow cytometer may have any number of lasers, beam splitters, filters, and detectors at various wavelengths and in various different configurations.

In operation, cytometer operation is controlled by a controller/processor **490,** and the measurement data from the detectors can be stored in the memory **495** and processed by the controller/processor **490.** Although not shown explicitly, the controller/processor **490** is coupled to the detectors to receive the output signals therefrom, and may also be coupled to electrical and electromechanical components of the flow cytometer **400** to control the lasers, fluid flow parameters, and the like. Input/output (I/O) capabilities **497** may be provided also in the system. The memory **495,** controller/processor **490,** and I/O **497** may be entirely provided as an integral part of the flow cytometer **410.** In such an embodiment, a display may also form part of the I/O capabilities **497** for presenting experimental data to users of the cytometer **400.** Alternatively, some or all of the memory **495** and controller/processor **490** and I/O capabilities may be part of one or more external devices such as a general purpose computer. In some embodiments, some or all of the memory **995** and controller/processor **490** can be in wireless or wired communication with the cytometer **410.** The controller/processor **490** in conjunction with the memory **495** and the I/O **497** can be configured to perform various functions related to the preparation and analysis of a flow cytometer experiment.

The system illustrated in **FIG. 9B** includes six different detectors that detect fluorescent light in six different wavelength bands (which may be referred to herein as a "filter window" for a given detector) as defined by the configuration of filters and/or splitters in the beam path from the flow cell **425** to each detector. Different fluorescent molecules used for a flow cytometer experiment will emit light in their own characteristic wavelength bands. The particular fluorescent labels used for an experiment and their associated fluorescent emission bands may be selected to generally coincide with the filter windows of the detectors. However, as more detectors are provided, and more labels are utilized, perfect correspondence between filter windows and fluorescent emission spectra is not possible. It is generally true that although the peak of the emission spectra of a particular fluorescent molecule may lie within the filter window of one particular detector, some of the emission spectra of that label will also overlap the filter windows of one or more other detectors. This may be referred to as spillover. The I/O **497** can be configured to receive data regarding a flow cytometer experiment having a panel of fluorescent labels and a plurality of cell populations having a plurality of markers, each cell population having a subset of the plurality of markers. The I/O **497** can also be configured to receive biological data assigning one or more markers to one or more cell populations, marker density data, emission spectrum data, data assigning labels to one or more markers, and cytometer configuration data. Flow cytometer experiment data, such as label spectral characteristics and flow cytometer configuration data can also be stored in the memory **495.** The controller/processor **490** can be configured to evaluate one or more assignments of labels to markers.

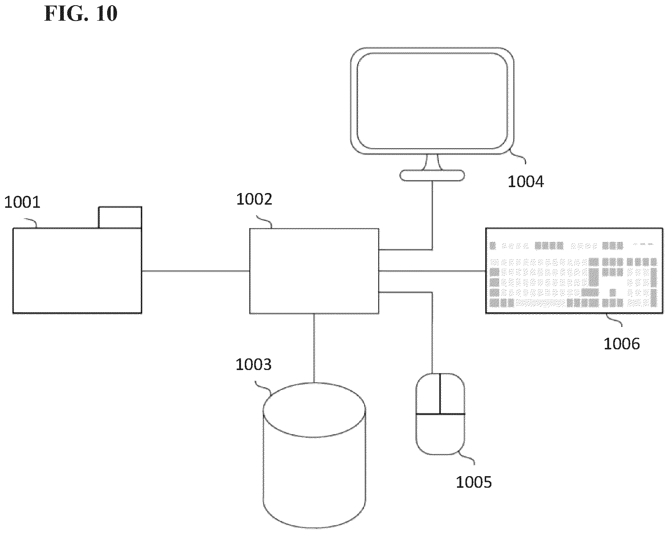

**FIG. 10** shows a functional block diagram for one example of a particle analyzer control system, such as an analytics controller **1002,** for analyzing and displaying biological events. An analytics controller **1002** can be configured to implement a variety of processes for controlling graphic display of biological events.

A particle analyzer or sorting system **1001** can be configured to acquire biological event data. For example, a flow cytometer can generate flow cytometric event data. The particle analyzer **1001** can be configured to provide biological event data to the analytics controller **1002.** A data communication channel can be included between the particle analyzer or sorting system **1001** and the analytics controller **1002.** The biological event data can be provided to the analytics controller **1002** via the data communication channel.

The analytics controller **1002** can be configured to receive biological event data from the particle analyzer or sorting system **1001.** The biological event data received from the particle analyzer or sorting system **1001** can include flow cytometric event data. The analytics controller **1002** can be configured to provide a graphical display including a first plot of biological event data to a display device **1004.** The analytics controller **1002** can be further configured to render a region of interest as a gate around a population of biological event data shown by the display device **1004,** overlaid upon the first plot, for example. In some embodiments, the gate can be a logical combination of one or more graphical regions of interest drawn upon a single parameter histogram or bivariate plot. In some embodiments, the display can be used to display particle parameters or saturated detector data.

The analytics controller **1002** can be further configured to display the biological event data on the display device **1004** within the gate differently from other events in the biological event data outside of the gate. For example, the analytics controller **1002** can be configured to render the color of biological event data contained within the gate to be distinct from the color of biological event data outside of the gate. The display device **1004** can be implemented as a monitor, a tablet computer, a smartphone, or other electronic device configured to present graphical interfaces.

The analytics controller **1002** can be configured to receive a gate selection signal identifying the gate from a first input device. For example, the first input device can be implemented as a mouse **1005.** The mouse **1005** can initiate a gate selection signal to the analytics controller **1002** identifying the gate to be displayed on or manipulated via the display device **1004** (e.g., by clicking on or in the desired gate when the cursor is positioned there). In some implementations, the first device can be implemented as the keyboard **1006** or other means for providing an input signal to the analytics controller **1002** such as a touchscreen, a stylus, an optical detector, or a voice recognition system. Some input devices can include multiple inputting functions. In such implementations, the inputting functions can each be considered an input device. For example, as shown in **FIG. 10****,** the mouse **1005** can include a right mouse button and a left mouse button, each of which can generate a triggering event. The triggering event can cause the analytics controller **1002** to alter the manner in which the data is displayed, which portions of the data is actually displayed on the display device **1004,** and/or provide input to further processing such as selection of a population of interest for particle sorting.

In some embodiments, the analytics controller **1002** can be configured to detect when gate selection is initiated by the mouse **1005.** The analytics controller **1002** can be further configured to automatically modify plot visualization to facilitate the gating process. The modification can be based on the specific distribution of biological event data received by the analytics controller **1002.**

The analytics controller **1002** can be connected to a storage device **1003.** The storage device **1003** can be configured to receive and store biological event data from the analytics controller **1002.** The storage device **1003** can also be configured to receive and store flow cytometric event data from the analytics controller **1002.** The storage device **1003** can be further configured to allow retrieval of biological event data, such as flow cytometric event data, by the analytics controller **1002.**

A display device **1004** can be configured to receive display data from the analytics controller **1002.** The display data can comprise plots of biological event data and gates outlining sections of the plots. The display device **1004** can be further configured to alter the information presented according to input received from the analytics controller **1002** in conjunction with input from the particle analyzer **1001,** the storage device **1003,** the keyboard **1006,** and/or the mouse **1005.**

In some implementations, the analytics controller **1002** can generate a user interface to receive example events for sorting. For example, the user interface can include a control for receiving example events or example images. The example events or images or an example gate can be provided prior to collection of event data for a sample, or based on an initial set of events for a portion of the sample.

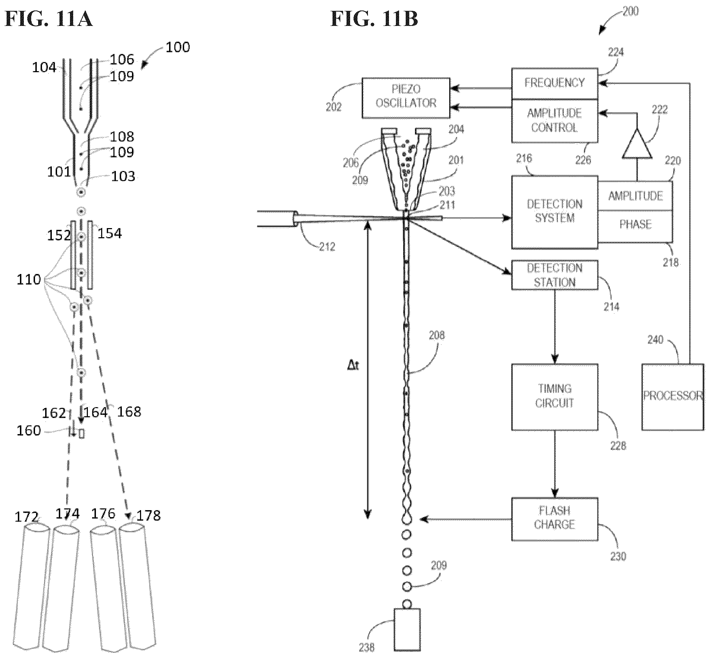

**FIG. 11A** is a schematic drawing of a particle sorter system **200** (e.g., the particle analyzer or sorting system **202)** in accordance with one embodiment presented herein. In some embodiments, the particle sorter system **200** is a cell sorter system. As shown in **FIG. 11A****,** a drop formation transducer **202** (e.g., piezo-oscillator) is coupled to a fluid conduit **201,** which can be coupled to, can include, or can be, a nozzle **203.** Within the fluid conduit **201,** sheath fluid **204** hydrodynamically focuses a sample fluid **206** comprising particles **209** into a moving fluid column **208** (e.g., a stream). Within the moving fluid column **208,** particles **209** (e.g., cells) are lined up in single file to cross a monitored area **211** (e.g., where laser-stream intersect), irradiated by an irradiation source **212** (e.g., a laser). Vibration of the drop formation transducer **202** causes moving fluid column **208** to break into a plurality of drops **210,** some of which contain particles **209.**

In operation, a detection station **214** (e.g., an event detector) identifies when a particle of interest (or cell of interest) crosses the monitored area **211.** Detection station **214** feeds into a timing circuit **228,** which in turn feeds into a flash charge circuit **230.** At a drop break off point, informed by a timed drop delay (Δt), a flash charge can be applied to the moving fluid column **208** such that a drop of interest carries a charge. The drop of interest can include one or more particles or cells to be sorted. The charged drop can then be sorted by activating deflection plates (not shown) to deflect the drop into a vessel such as a collection tube or a multi- well or microwell sample plate where a well or microwell can be associated with drops of particular interest. As shown in **FIG. 11A****,** the drops can be collected in a drain receptacle **238.**

A detection system **216** (e.g., a drop boundary detector) serves to automatically determine the phase of a drop drive signal when a particle of interest passes the monitored area **211.** An exemplary drop boundary detector is described in U.S. Pat. No. 7,679,039. , . The detection system **616** allows the instrument to accurately calculate the place of each detected particle in a drop. The detection system **216** can feed into an amplitude signal **220** and/or phase **218** signal, which in turn feeds (via amplifier **222)** into an amplitude control circuit **226** and/or frequency control circuit **224.** The amplitude control circuit **226** and/or frequency control circuit **224,** in turn, controls the drop formation transducer **202.** The amplitude control circuit **226** and/or frequency control circuit **224** can be included in a control system.

In some implementations, sort electronics (e.g., the detection system **216,** the detection station **214** and a processor **240)** can be coupled with a memory configured to store the detected events and a sort decision based thereon. The sort decision can be included in the event data for a particle. In some implementations, the detection system **216** and the detection station **214** can be implemented as a single detection unit or communicatively coupled such that an event measurement can be collected by one of the detection system **216** or the detection station **214** and provided to the non-collecting element.

**FIG. 11B** is a schematic drawing of a particle sorter system, in accordance with one embodiment presented herein. The particle sorter system **100** shown in **FIG. 11B****,** includes deflection plates **152** and **154.** A charge can be applied via a stream-charging wire in a barb. This creates a stream of droplets **110** containing particles **110** for analysis. The particles can be illuminated with one or more light sources (e.g., lasers) to generate light scatter and fluorescence information. The information for a particle is analyzed such as by sorting electronics or other detection system (not shown in **FIG. 11B****).** The deflection plates **152** and **154** can be independently controlled to attract or repel the charged droplet to guide the droplet toward a destination collection receptacle (e.g., one of **172, 174, 176,** or **178).** As shown in **FIG. 11B****,** the deflection plates **152** and **154** can be controlled to direct a particle along a first path **162** toward the receptacle **174** or along a second path **168** toward the receptacle **178.** If the particle is not of interest (e.g., does not exhibit scatter or illumination information within a specified sort range), deflection plates may allow the particle to continue along a flow path **164.** Such uncharged droplets may pass into a waste receptacle such as via aspirator **170.**

The sorting electronics can be included to initiate collection of measurements, receive fluorescence signals for particles, and determine how to adjust the deflection plates to cause sorting of the particles. Example implementations of the embodiment shown in **FIG. 11B** include the BD FACSAria^{™} line of flow cytometers commercially provided by Becton, Dickinson and Company (Franklin Lakes, NJ).

In some embodiments, particle sorting systems of interest are configured to sort particles with an enclosed particle sorting module, such as those described in U.S. Patent Publication No. 2017/0299493, filed on March 28, 2017.In certain embodiments, particles (e.g., cells) of the sample are sorted using a sort decision module having a plurality of sort decision units, such as those described in U.S. Patent Publication No. 2020/0256781.. In some embodiments, the subject systems include a particle sorting module having deflector plates, such as described in U.S. Patent Publication No. 2017/0299493, filed on March 28, 2017.

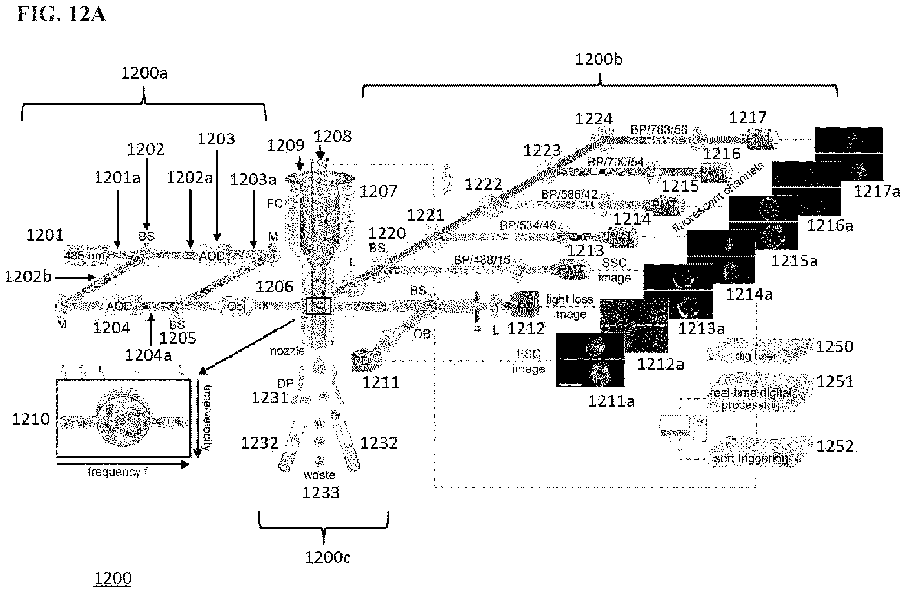

In certain embodiments, systems are a fluorescence imaging using radiofrequency tagged emission image-enabled particle sorter, such as depicted in **FIG. 12A****.** Particle sorter **1200** includes a light irradiation component **1200a** which includes light source **1201** (e.g., 488 nm laser) which generates output beam of light **1201a** that is split with beamsplitter **1202** into beams **1202a** and **1202b.** Light beam **1202a** is propagated through acousto-optic device (e.g., an acousto-optic deflector, AOD) **1203** to generate an output beam **1203a** having one or more angularly deflected beams of light. In some instances, output beam **1203a** generated from acousto-optic device **1203** includes a local oscillator beam and a plurality of radiofrequency comb beams. Light beam **1202b** is propagated through acousto-optic device (e.g., an acousto-optic deflector, AOD) **1204** to generate an output beam **1204a** having one or more angularly deflected beams of light. In some instances, output beam **1204a** generated from acousto-optic device **1204** includes a local oscillator beam and a plurality of radiofrequency comb beams. Output beams **1203a** and **1204a** generated from acousto-optic devices **1203** and **1204,** respectively are combined with beamsplitter **1205** to generate output beam **1205a** which is conveyed through an optical component **1206** (e.g., an objective lens) to irradiate particles in flow cell **1207.** In certain embodiments, acousto-optic device **1203** (AOD) splits a single laser beam into an array of beamlets, each having different optical frequency and angle. Second AOD **1204** tunes the optical frequency of a reference beam, which is then overlapped with the array of beamlets at beam combiner **1205.** In certain embodiments, the light irradiation system having a light source and acousto-optic device can also include those described in Schraivogel, et al. ("High-speed fluorescence image-enabled cell sorting," Science (2022), 375 (6578): 305-320) and United States Patent Publication No. 2021/0404943..

Output beam **1205a** irradiates sample particles **1208** propagating through flow cell **1207** (e.g., with sheath fluid **1209)** at irradiation region **1210.** As shown in irradiation region **1210,** a plurality of beams (e.g., angularly deflected radiofrequency shifted beams of light depicted as dots across irradiation region **1210)** overlaps with a reference local oscillator beam (depicted as the shaded line across irradiation region **1210).** Due to their differing optical frequencies, the overlapping beams exhibit a beating behavior, which causes each beamlet to carry a sinusoidal modulation at a distinct frequency f₁₋ₙ.

Light from the irradiated sample is conveyed to light detection system **1200b** that includes a plurality of photodetectors. Light detection system **1200b** includes forward scattered light photodetector **1211** for generating forward scatter images **1211a** and a side scattered light photodetector **1212** for generating side scatter images **1212a.** Light detection system **1200b** also includes brightfield photodetector **1213** for generating light loss images **1213a.** In some embodiments, forward scatter detector **1211** and side scatter detector **1212** are photodiodes (e.g., avalanche photodiodes, APDs). In some instances, brightfield photodetector **1213** is a photomultiplier tube (PMT). Fluorescence from the irradiated sample is also detected with fluorescence photodetectors **1214-1217.** In some instances, photodetectors **1214-1217** are photomultiplier tubes. Light from the irradiated sample is directed to the side scatter detection channel **1212** and fluorescence detection channels **1214-1217** through beamsplitter **1220.** Light detection system **1200b** includes bandpass optical components **1221, 1222, 1223** and **1224** (e.g., dichroic mirrors) for propagating predetermined wavelength of light to photodetectors **1214-1217.** In some instances, optical component **1221** is a 534 nm/40 nm bandpass. In some instances, optical component **1222** is a 586 nm/42 nm bandpass. In some instances, optical component **1223** is a 700 nm/54 nm bandpass. In some instances, optical component **1224** is a 783 nm/56 nm bandpass. The first number represents the center of a spectral band. The second number provides a range of the spectral band. Thus, a 510/20 filter extends 10 nm on each side of the center of the spectral band, or from 500 nm to 520 nm.

Data signals generated in response to light detected in scattered light detection channels **1211** and **1212,** brightfield light detection channel **1213** and fluorescence detection channels **1214-1217** are processed by real-time digital processing with processors **1250** and **1251.** Images **1211a-1217a** can be generated in each light detection channel based on the data signals generated in processors **1250** and **1251.** Image-enabled sorting is performed in response to a sort signal generated in sort trigger **1252.** Sorting component **1200c** includes deflection plates **1231** for deflecting particles into sample containers **1232** or to waste stream **1233.** In some instances, sort component **1200c** is configured to sort particles with an enclosed particle sorting module, such as those described in U.S. Patent Publication No. 2017/0299493, filed on March 28, 2017. In certain embodiments, sorting component **1200c** includes a sort decision module having a plurality of sort decision units, such as those described in U.S. Patent Publication No. 2020/0256781.

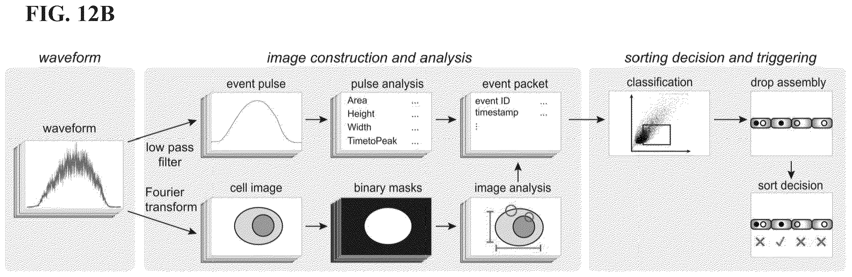

Figure 12B depicts image-enabled particle sorting data processing according to certain embodiments. In some instances, image-enabled particle sorting data processing is a low-latency data processing pipeline. Each photodetector produces a pulse with high-frequency modulations encoding the image (waveform). Fourier analysis is performed to reconstruct the image from the modulated pulse. An image processing pipeline produces a set of image features (image analysis), which are combined with features derived from a pulse processing pipeline (event packet). Real-time sort classification electronics then classify the particle based on image features, producing a sort decision that is used to selectively charge the droplets.

### COMPUTER-CONTROLLED SYSTEMS

Aspects of the present disclosure further include computer-controlled systems, where the systems include one or more computers for complete automation or partial automation of the subject methods and systems of particle analysis. In some embodiments, systems include a computer with a computer program stored thereon, where the computer program when loaded on the computer includes instructions for displaying data in accordance with embodiment of the invention. In some instances, the system is, or comprises, a flow cytometer.

Systems may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor, or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, Python, other high level or low level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. In some embodiments, the processor includes analog electronics which provide feedback control, such as for example negative feedback control.

The system memory may be any of a variety of known or future memory storage devices. Examples include any commonly available random access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, or a diskette drive. Such types of memory storage devices typically read from, and/or write to, a program storage medium (not shown) such as a compact disk. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

In some embodiments, a computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. Systems of the invention also include programming, e.g., in the form of computer program products, algorithms for use in practicing the methods as described above. Programming according to the present invention can be recorded on computer readable media, e.g., any medium that can be read and accessed directly by a computer. Such media include, but are not limited to: magnetic storage media, optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a computer connected to a Wide Area Network ("WAN"), telephone network, satellite network, or any other suitable communication channel, including a mobile telephone (i.e., smartphone).

In some embodiments, systems according to the present disclosure may be configured to include a communication interface. In some embodiments, the communication interface includes a receiver and/or transmitter for communicating with a network and/or another device. The communication interface can be configured for wired or wireless communication, including, but not limited to, radio frequency (RF) communication (e.g., Radio-Frequency Identification (RFID), Zigbee communication protocols, Wi-Fi, infrared, wireless Universal Serial Bus (USB), Ultra-Wide Band (UWB), Bluetooth^{®} communication protocols, and cellular communication, such as code division multiple access (CDMA) or Global System for Mobile communications (GSM).

In one embodiment, the communication interface is configured to include one or more communication ports, e.g., physical ports or interfaces such as a USB port, a USB-C port, an RS-232 port, or any other suitable electrical connection port to allow data communication between the subject systems and other external devices such as a computer terminal (for example, at a physician's office or in hospital environment) that is configured for similar complementary data communication.

In one embodiment, the communication interface is configured for infrared communication, Bluetooth^{®} communication, or any other suitable wireless communication protocol to enable the subject systems to communicate with other devices such as computer terminals and/or networks, communication enabled mobile telephones, personal digital assistants, or any other communication devices which the user may use in conjunction.

In one embodiment, the communication interface is configured to provide a connection for data transfer utilizing Internet Protocol (IP) through a cell phone network, Short Message Service (SMS), wireless connection to a personal computer (PC) on a Local Area Network (LAN) which is connected to the internet, or Wi-Fi connection to the internet at a Wi-Fi hotspot.

In one embodiment, the subject systems are configured to wirelessly communicate with a server device via the communication interface, e.g., using a common standard such as 802.11 or Bluetooth^{®} RF protocol, or an IrDA infrared protocol. The server device may be another portable device, such as a smart phone, Personal Digital Assistant (PDA) or notebook computer; or a larger device such as a desktop computer, appliance, etc. In some embodiments, the server device has a display, such as a liquid crystal display (LCD), as well as an input device, such as buttons, a keyboard, mouse or touch-screen.

In some embodiments, the communication interface is configured to automatically or semiautomatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a workstation, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows^{®} NTO, Windows^{®} XP, Windows^{®} 7, Windows^{®} 8, Windows^{®} 10, iOS^{®}, macOSO, Linux^{®}, Ubuntu^{®}, Fedora^{®}, OS/400^{®}, i5/OSO, IBM i^{®}, Android^{™}, SGI IRIXO, Oracle Solaris^{®} and others.

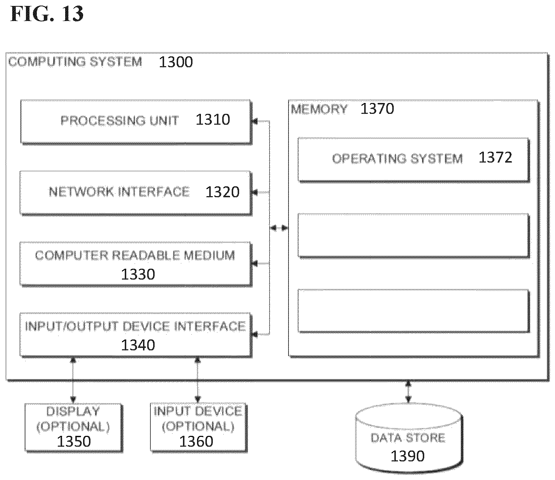

**FIG. 13** depicts a general architecture of an example computing device **1300** according to certain embodiments. The general architecture of the computing device **1300** depicted in **FIG. 13** includes an arrangement of computer hardware and software components. It is not necessary, however, that all of these generally conventional elements be shown in order to provide an enabling disclosure. As illustrated, the computing device **1300** includes a processing unit **1310,** a network interface **1320,** a computer readable medium drive **1330,** an input/output device interface **1340,** a display **1350,** and an input device **1360,** all of which may communicate with one another by way of a communication bus. The network interface **1320** may provide connectivity to one or more networks or computing systems. The processing unit **1310** may thus receive information and instructions from other computing systems or services via a network. The processing unit **1310** may also communicate to and from memory **1370** and further provide output information for an optional display **1350** via the input/output device interface **1340.** For example, an analysis software (e.g., data analysis software or program such as FlowJo^{®}) stored as executable instructions in the non-transitory memory of the analysis system can display the flow cytometry event data to a user. The input/output device interface **1340** may also accept input from the optional input device **1360,** such as a keyboard, mouse, digital pen, microphone, touch screen, gesture recognition system, voice recognition system, gamepad, accelerometer, gyroscope, or other input device.

The memory **1370** may contain computer program instructions (grouped as modules or components in some embodiments) that the processing unit **1310** executes in order to implement one or more embodiments. The memory **1370** generally includes RAM, ROM and/or other persistent, auxiliary or non-transitory computer-readable media. The memory **1370** may store an operating system **1372** that provides computer program instructions for use by the processing unit **1310** in the general administration and operation of the computing device **1300.** Data may be stored in data storage device **1390.** The memory **1370** may further include computer program instructions and other information for implementing aspects of the present disclosure.

### COMPUTER-READABLE STORAGE MEDIA

Aspects of the present disclosure further include non-transitory computer readable storage mediums having instructions for practicing the subject methods. Computer readable storage mediums may be employed on one or more computers for complete automation or partial automation of a system for practicing methods described herein. In certain embodiments, instructions in accordance with the method described herein can be coded onto a computer-readable medium in the form of "programming", where the term "computer readable medium" as used herein refers to any non-transitory storage medium that participates in providing instructions and data to a computer for execution and processing. In some cases, the instructions, when executed by a computer or processor, cause the computer or processor to receive display data in accordance with embodiments of the invention.

Examples of suitable non-transitory storage media include a floppy disk, hard disk, optical disk, magneto-optical disk, CD-ROM, CD-R, magnetic tape, non-volatile memory card, ROM, DVD-ROM, Blue-ray disk, solid state disk, flash drive, and network attached storage (NAS), whether or not such devices are internal or external to the computer. A file containing information can be "stored" on computer readable medium, where "storing" means recording information such that it is accessible and retrievable at a later date by a computer. The computer-implemented method described herein can be executed using programming that can be written in one or more of any number of computer programming languages. Such languages include, for example, Java, Python, Visual Basic, and C++, as well as many others.

### KITS

Aspects of the present disclosure also include kits. The kits may include, e.g., a compensation control composition as described herein. In certain embodiments, the control composition of the kit may include a positive control particle as described herein. In some embodiments, the control composition of the kit may include a negative control particle as described herein. In some cases, the control composition of the kit may include a plurality of the positive control particles and/or a plurality of the negative control particles.

The kits may further include an amine reactive dye and/or a DNA binding dye for labeling the positive control particles of the compensation control composition. In some embodiments, the kits may further include a buffer. For instance, the kit may include a buffer, such as a sample buffer, a wash buffer, an assay buffer, and the like. The kits may further include additional reagents such as, but not limited to, fluorescent particles and detectable labels (e.g., fluorescent labels, colorimetric labels, chemiluminescent labels, multicolor reagents, avidin-streptavidin associated detection reagents, radiolabels, gold particles, magnetic labels, etc.), and the like.

In certain embodiments, the kit includes a subject compensation control composition and a packaging configured to hold the composition. The packaging may be a sealed packaging, e.g., a water vapor-resistant container, optionally under an air-tight and/or vacuum seal. In some embodiments, the packaging may protect its internal contents (e.g., the subject compositions) from light. In these instances, the packaging may protect against any range or spectrum of light wavelengths including, e.g., visible light, UV light, light in and/or near the excitation spectrum of any enclosed dyes, etc. In certain instances, the packaging is a sterile packaging, configured to maintain the compositions enclosed in the packaging in a sterile environment. By "sterile" is meant that there are substantially no microbes (such as fungi, bacteria, viruses, spore forms, etc.). In some embodiments, the compositions, and any further included buffers or reagents (e.g., amine reactive dyes and/or a DNA binding dyes), are included in the same packaging. In other instances, the compositions, and any further included buffers or reagents, are included in separate packaging.

In addition to the above components, the subject kits may further include instructions for practicing the subject methods. In some embodiments, the instructions are for using the compensation control composition of the kit to determine a compensation value for cell viability data obtained from a flow cytometry analysis. In some embodiments, the instructions are for performing the flow cytometry analysis and using the determined compensation value to perform compensation on the obtained cell viability data. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Another means would be a computer readable medium (e.g., as described above), on which the information has been recorded or stored. Yet another form that may be present is a website address which may be used via the Internet to access the information at a removed site. Any convenient form of instructions may be present in the kits.

### UTILITY

The subject compositions and methods find use in applications where cell analysis of a biological sample may be desired for research, laboratory testing, or for use in therapy. In some embodiments, the subject systems and methods facilitate analysis of cells obtained from fluidic or tissue samples such as, e.g., specimens for diseases, including but not limited to cancer. The compositions and methods of the present disclosure also allow for analyzing cells from a biological sample (e.g., organ, tissue, tissue fragment, fluid) with enhanced efficiency and at a low cost.

The subject control compositions and methods find use in applications where the analysis of a sample using ultraviolet (UV) or violet lasers is desired. Embodiments of the subject control compositions and methods find use where the analysis of a sample using two or more fluorescent particles (e.g., two or more dye compositions) is desired. For example, the subject compositions and methods find use in applications where it is desired to perform an assay using an amine reactive dye and a DNA binding dye (such as, e.g., a cell viability assay). In some cases, the subject compositions and methods allow for fluorescence compensation to be performed for assays including one or more of: amine reactive dyes, DNA binding dyes, fluorescently tagged nucleic acid probes, and fluorescently tagged antibodies.

The subject control compositions and methods find use where it is desired to perform flow cytometry assays having more precise and/or accurate data. For instance, the subject compositions and methods may facilitate accurate data collection and interpretation as compared to protocols and control beads of the prior art having positive compensation controls of a lower fluorescence intensity or negative control samples of a higher fluorescence intensity. Further, the subject control compositions and methods may facilitate accurate data collection and interpretation as compared to prior art protocols and control beads unable to include fluorescence minus one (FMO) controls for assays including two or more of: amine reactive dyes, DNA binding dyes, fluorescently tagged nucleic acid probes, and fluorescently tagged antibodies.

The subject compositions and methods find use in laboratory and research workflows desiring a high degree of flexibility. For instance, the subject compositions and methods may facilitate the ability of a laboratory to run customizable experiments on a wide array of different cell types. Further, the subject compositions and methods may facilitate a reduction in cost as compared to the less stable control beads of the prior art. Embodiments of the subject compositions and methods allow fluorescence compensation to be performed without any of a cell sample being depleted for use as a control. Thus, the subject compositions and methods further find use where it is desired to analyze as much of a collected cellular sample as possible, e.g., due to low sample availability, to save cost, or due to difficulties that may exist in sample collection.

### EXAMPLES

As can be appreciated from the disclosure provided above, embodiments of the present disclosure have a wide variety of applications. Accordingly, the following examples are put forth so as to provide those of ordinary skill in the art with a complete disclosure and description of how to make and use the present invention, and are not intended to limit the scope of what the inventors regard as their invention, nor are they intended to represent that the experiments below are all or the only experiments performed. Efforts have been made to ensure accuracy with respect to numbers used (e.g., amounts, temperature, etc.) but some experimental errors and deviations should be accounted for. Unless indicated otherwise, temperature is in degrees Celsius, and pressure approximately atmospheric. In addition, common laboratory protocol abbreviations may be used (e.g., µl = microliters, min = minutes, kcal = kilocalorie, mol = moles, etc.).

The following experiments demonstrate the stability of cell viability compensation (CVC) beads of the present invention and compare positive and negative CVC beads with prior art compensation beads.

### Materials and Methods

An embodiment of a compensation control composition having both positive and negative control beads in accordance with embodiments of the invention was produced. The positive control beads were synthesized in accordance with embodiments of the invention and included: a silica core, 5 layers of a histone protein coating, and 5 layers of a dsDNA molecule coating. For the positive control, a dsDNA coating was first covalently attached to the core. Histone layers of coating and dsDNA layers of coating were then covalently attached to the outermost coating of the positive control bead in an alternating manner until there were 5 layers of each. The negative control beads were synthesized in accordance with embodiments of the invention and included a silica core, but lacked both aminated polymer (i.e., histone) coatings and nucleic acid (i.e., dsDNA) coatings.

**FIG. 3** provides a depiction of the method used to synthesize the positive control bead. An aminated silica core was obtained, and a plurality of its amine groups were thiolated. Coatings of dsDNA molecules modified to include maleimide groups and coatings of histone proteins modified to include thiol groups were then alternately deposited onto the positive control bead until there were 5 layers of each.

### Example 1: Staining with Amine Reactive Dyes

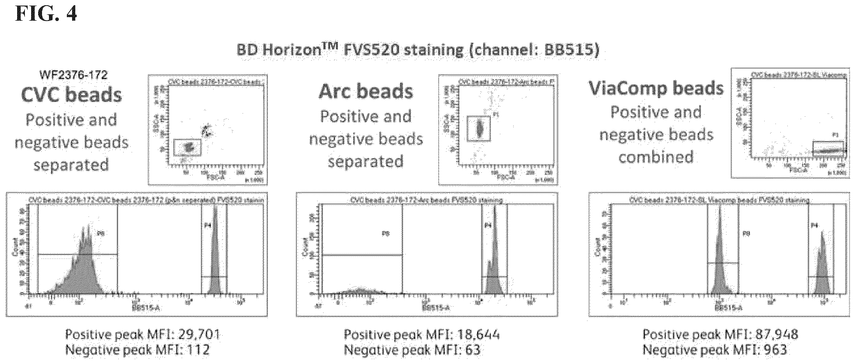

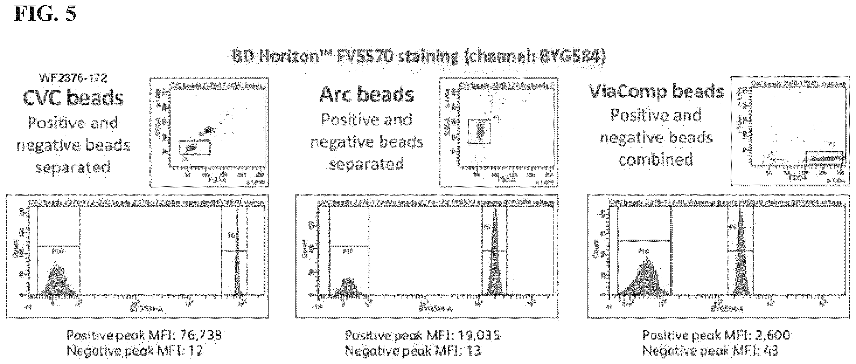

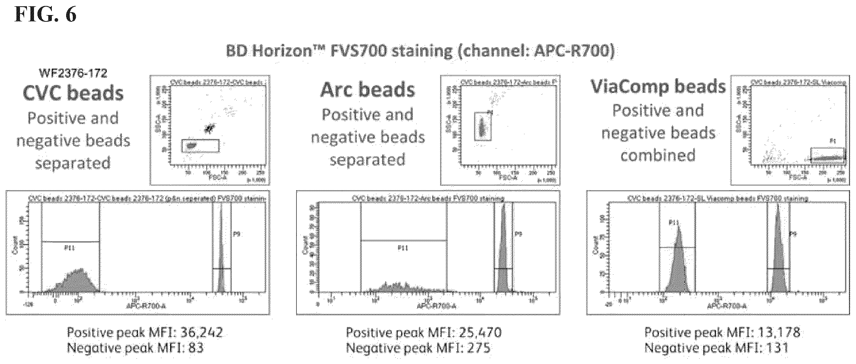

Experiments were performed to compare fluorescence resulting from amine reactive dye staining between an embodiment of the CVC beads of the present invention (see "Materials and Methods" above) and two types of beads of the prior art: ViaComp^{®} (Slingshot) beads and Arc^{™} (Invitrogen) beads. The ViaComp^{®} (Slingshot) beads are composed of a hydrogel and the Arc^{™} (Invitrogen) beads are specially modified polystyrene microspheres. Three different BD Horizon^{™} Fixable Viability Stains (FVS) amine reactive dyes were used for staining: BD Horizon^{™} FVS520 **(****FIG. 4****)** (peak excitation: 498 nm; peak emission: 521 nm; fluorescence channel: BB515), BD Horizon^{™} FVS570 **(****FIG. 5****)** (peak excitation: 547 nm; peak emission: 573 nm; fluorescence channel: BYG584), and BD Horizon^{™} FVS700 **(****FIG. 6****)** (peak excitation: 657 nm; peak emission: 700 nm; fluorescence channel: APC-R700). For staining, 50 µl of bead suspension (i.e., both positive and negative beads) and 1 µl of FVS dye was incubated in DMSO buffer for 30 min. Data was collected under the same flow cytometer parameter settings.

The data presented in **FIGS. 4-6** demonstrate that the CVC beads of the present invention exhibit either brighter or comparable staining signa when compared to the prior art ViaComp^{®} and Arc^{™} beads. However, preliminary experiments have shown that the fluorescence intensity signal of the stained CVC beads of the present invention may be tuned or adjusted (e.g., increased) by controlling the number of layers of the histone coating. Further, unlike the CVC beads, the polystyrene of the Arc^{™} beads exhibited significant autofluorescence, resulting in different spectral signatures of the beads after being stained by the cell viability dyes when compared to stained cell samples. This problem was especially notable for fluorescence emission by the violet and ultraviolet lasers. Such significant autofluorescence causes misleading spillover values for dyes, especially dyes emitting or absorbing light in the UV and violet ranges, potentially compromising the compensation data of experiments.

The CVC beads of the present invention also demonstrated logistical advantages over the Arc^{™} beads. Unlike embodiments of the CVC beads, the Arc^{™} beads were split into two vials: one vial of positive beads and one vial of negative control beads. This division results in added complexity and production costs and, further, creates an inconvenience for users during the experimental process. Additionally, the Arc^{™} beads can only be stained by amine-reactive dyes as they have no reactivity with DNA-intercalating dyes. This limits the options of users when designing experiments.

### Example 2: Staining with DNA Binding Dyes

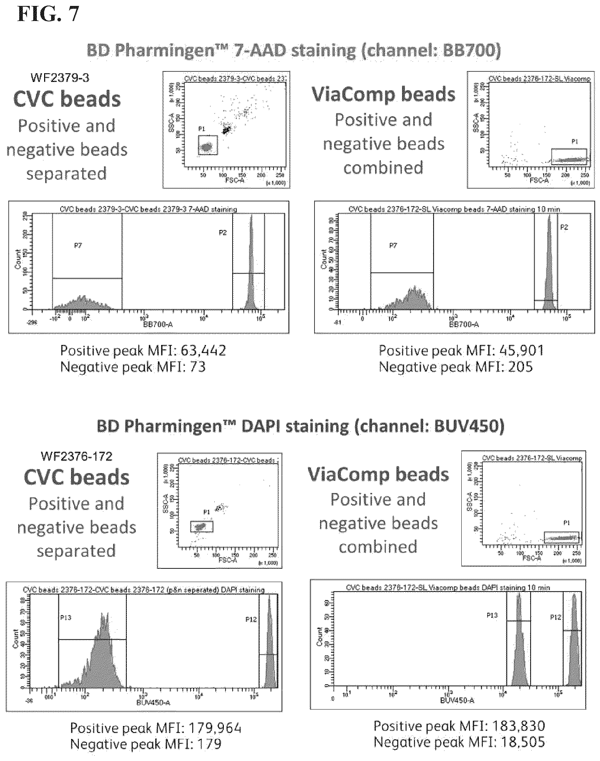

Experiments were performed to compare fluorescence resulting from DNA-intercalating dye staining between an embodiment of the CVC beads of the present invention (see "Materials and Methods" above) and the ViaComp^{®} (Slingshot) hydrogel beads of the prior art. Two different BD Pharmingen^{™} DNA-intercalating dyes were used for staining: BD Pharmingen^{™} 7-AAD (7-Amino-Actinomycin D) **(****FIG. 7****)** (fluorescence channel: BB700) and BD Pharmingen^{™} DAPI (4',6-diamidino-2-phenylindole) **(****FIG. 7****)** (fluorescence channel: BUV450). For staining, 50 µl of bead suspension (i.e., both positive and negative beads) and either 20 µl of BD Pharmingen^{™} 7-AAD or 1 µl of BD Pharmingen^{™} DAPI was incubated in solution for 10 min. Data was collected under the same flow cytometer parameter settings.

The data presented in **FIG. 7** demonstrates that the CVC beads of the present invention exhibit either brighter or comparable staining signa when compared to the prior art ViaComp^{®} beads. However, preliminary experiments have shown that the fluorescence intensity signal of the stained CVC beads of the present invention may be tuned or adjusted (e.g., increased) by controlling the number of layers of the dsDNA coating. Further, unlike the CVC beads, high non-specific uptake of DNA-intercalation dyes on the ViaComp^{®} negative control beads was observed. This non-specific uptake, likely due to the hydrogel composition of the beads, leads to high background noise - potentially lowering the accuracy of flow cytometry results obtained using the ViaComp^{®} beads.

### Example 3: Control Composition Stability

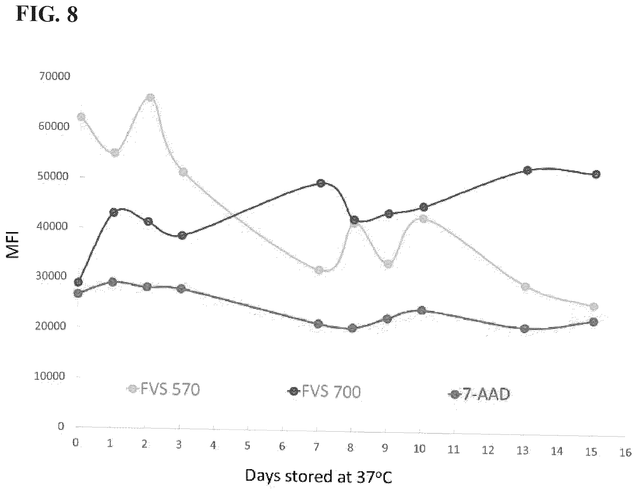

Experiments were performed to test the stability of an embodiment of the CVC beads of the present invention (see "Materials and Methods" above). The CVC beads were stored at 37°C for 15 days, with subsets of the beads being tested on days 1-3, 7-10, 13 and 15. To test, beads were stained using either BD Pharmingen^{™} 7-AAD, BD Horizon^{™} FVS570, or BD Horizon^{™} FVS700 and the fluorescence intensity of beads stained with each dye was measured. The MFIs of beads stained with each of the dyes on each of the tested days can be found in **FIG. 8****.**

The data presented in **FIG. 8** demonstrates that the CVC beads of the present invention can be stored at relatively high temperatures for several weeks without a significant loss of dye staining performance. Using the Arrhenius equation (Q₁₀ = 3, Eₐ =19.4 kcal/mol), it was predicted that the CVC beads could still be efficiently stained by both amine reactive and DNA intercalating dyes after 18 months of storage at 4°C. Accordingly, the CVC beads demonstrate a significant advantage over prior art beads such as, e.g., ViaComp^{®} beads, which only have a shelf life of six months at 2-8°C and, as such, must be shipped and stored at -20°C. Such temperature instability adds inconvenience to users, as beads must be thawed prior to use and expired beads must be replaced - leading to additional costs.

Notwithstanding the appended claims, the disclosure is also defined by the following clauses:
1. A positive control particle comprising:
   a core;
   an aminated polymer comprising an amine configured to react with an amine reactive dye; and
   a nucleic acid configured to bind to a DNA binding dye;
   wherein the aminated polymer and the nucleic acid are covalently associated with the core.
2. The positive control particle according to Clause 1, wherein the core is substantially non-autofluorescent.
3. The positive control particle according to Clauses 1 or 2, wherein the core has low non-specific binding.
4. The positive control particle according to any of the preceding clauses, wherein the core comprises an inorganic material.
5. The positive control particle according to Clause 4, wherein the core comprises silica.
6. The positive control particle according to any of Clauses 1 to 3, wherein the core comprises a polymer.
7. The positive control particle according to Clause 6, wherein the core comprises poly(methyl methacrylate) (PMMA).
8. The positive control particle according to Clause 6, wherein the core comprises polyacrylamide hydrogel.
9. The positive control particle according to any of the preceding clauses, wherein the particle has a diameter ranging from 0.1 to 150 microns.
10. The positive control particle according to Clause 9, wherein the particle has a diameter ranging from 1 to 10 microns.
11. The positive control particle according to Clause 10, wherein the particle has a diameter ranging from 5 to 7 microns.
12. The positive control particle according to any of the preceding clauses, wherein the aminated polymer and nucleic acid are layered on the core.
13. The positive control particle according to Clause 12, wherein the particle comprises 5 or more aminated polymer layers and 5 or more nucleic acid layers.
14. The positive control particle according to any of the preceding clauses, wherein the aminated polymer comprises a polypeptide.
15. The positive control particle of clause 14, wherein the polypeptide is a protein.
16. The positive control particle of clause 15, wherein the protein is a histone.
17. The positive control particle of clause 15, wherein the protein is a myelin basic protein.
18. The positive control particle according to any of Clauses 1 to 14, wherein the aminated polymer comprises a polysaccharide.
19. The positive control particle according to Clause 18, wherein polysaccharide is a cationic polymer.
20. The positive control particle according to Clause 19, wherein the cationic polymer is chitosan.
21. The positive control particle according to any of the preceding clauses, wherein the nucleic acid is a double-stranded nucleic acid.
22. The positive control particle according to any of the preceding clauses, wherein the nucleic acid is a deoxyribonucleic acid (DNA).
23. The positive control particle according to any of the preceding clauses, wherein the nucleic acid has size ranging from 450 to 550 base pairs.
24. The positive control particle according to any of the preceding clauses, wherein the nucleic acid is naturally occurring.
25. The positive control particle according to Clause 24, wherein the nucleic acid comprises salmon DNA.
26. The positive control particle according to any of Clauses 1 to 23, wherein the nucleic acid is synthetic.
27. The positive control particle according to any of the preceding clauses, wherein the aminated polymer and the nucleic acid are covalently bound directly or through a linker to a surface of the core.
28. The positive control particle according to any of the preceding clauses, wherein the particle is a bead.
29. A compensation control composition comprising a plurality of positive control particles according to any of the preceding clauses.
30. The compensation control composition according to Clause 29, further comprising a plurality of negative control particles.
31. The compensation control composition according to Clause 30, wherein the negative control particles comprise a core that comprises the same material and the same average diameter as the core of the positive control particles.
32. The compensation control composition according to Clauses 30 or 31, wherein a polyethylene glycol (PEG) is conjugated to the core of the negative control particles.
33. The compensation control composition according to any of Clauses 29 to 32, wherein the composition is stable at 4 degrees Celsius for 18 or more months.
34. The compensation control composition of Clause 1, further comprising the amine reactive dye.
35. A labeled compensation control composition comprising a plurality of positive control particles according to any of Clauses 1 to 28, wherein the positive control particles have an amine reactive dye and/or a DNA binding dye bound thereto.
36. The labeled compensation control composition according to Clause 35, wherein the amine reactive dye is a cell viability dye.
37. The labeled compensation control composition according to Clauses 35 or 36, wherein the amine reactive dye has an excitation maximum ranging from 350 nm to 1000 nm.
38. The labeled compensation control composition according to any of Clauses 35 to 37, wherein the amine reactive dye is selected from the group consisting of a BD Horizon^{™} Fixable Viability Stain, a Biolegend Zombie^{™} dye, a Thermo Fischer eFluor^{™} dye, a Thermo Fischer LIVE/DEAD^{™} stain, a Proteintech Phantom Dye and a Tonbo Biosceinces Ghost Dye^{™}.
39. The labeled compensation control composition according to any of Clauses 35 to 38, wherein the DNA binding dye is a cell viability dye.
40. The labeled compensation control composition according to any of Clauses 35 to 39, wherein the DNA binding dye has an excitation maximum ranging from 350 nm to 1000 nm.
41. The labeled compensation control composition according to any of Clauses 35 to 40, wherein the DNA binding dye is selected from the group consisting of 7-AAD, DAPI, propidium iodide, a Hoechst dye, ethidium bromide, LDS 751, a Thermo Fischer Sytox^{™} dye, a Thermo Fischer T-PRO^{™} dye, a Thermo Fischer TOTO^{™} dye, a Thermo Fischer YO-PRO^{™} dye, a Biolegend Helix-NP^{™} dye, a Biotium RedDot^{™} dye and a Biostatus Limited DRAQ^{™} dye.
42. The labeled compensation control composition of according to any of Clauses 35 to 41, further comprising a plurality of negative control particles.
43. The labeled compensation control composition according to Clause 42, wherein the negative control particles comprise a core that comprises the same material and the same average diameter as the core of the positive control particles.
44. The labeled compensation control composition according to Clause 43, wherein a PEG is conjugated to the core of the negative control particles.
45. A method of determining a compensation value for cell viability data obtained from a flow cytometry analysis, the method comprising:
   analyzing a labeled compensation control composition according to any of Clauses 35 to 44 using a flow cytometer to obtain flow cytometry data; and
   calculating a compensation value based on the flow cytometry data.
46. The method according to Clause 45, further comprising preparing the labeled compensation control composition.
47. The method according to Clause 46, wherein preparing comprises contacting a compensation control composition according to any of Clauses 29 to 34 with an amine reactive dye and/or a DNA binding dye.
48. The method according to any of Clauses 45 to 47, further comprising performing compensation on cell viability data, the compensation comprising:
   contacting a cell sample with the same dye bound to the positive control particle,
   producing the cell viability data by analyzing the cell sample using a flow cytometer; and
   modifying the cell viability data using the calculated compensation value.
49. A method of producing a positive control particle, the method comprising:
   covalently associating an aminated polymer and nucleic acid with a core to produce a positive control particle.
50. The method according to Clause 49, wherein the aminated polymer and the nucleic acid are layered on a surface of the core.
51. A kit comprising:
   a compensation control composition according to any of Clauses 29 to 34.
52. The kit according to Clause 51, wherein the kit further comprises an amine reactive dye.
53. The kit according to any of Clauses 51 or 52, wherein the kit further comprises a DNA binding dye.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

It will be understood by those within the art that, in general, terms used herein, and especially in the appended claims (*e.g*., bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

## Claims

1. A positive control particle comprising:
a core;
an aminated polymer comprising an amine configured to react with an amine reactive dye; and
a nucleic acid configured to bind to a DNA binding dye;
wherein the aminated polymer and the nucleic acid are covalently associated with the core.

2. The positive control particle according to claim 1, wherein the core is substantially non-autofluorescent.

3. The positive control particle according to claims 1 or 2, wherein the core has low non-specific binding.

4. The positive control particle according to any of the preceding claims, wherein the core comprises an inorganic material.

5. The positive control particle according to any of the preceding claims, wherein the aminated polymer and nucleic acid are layered on the core.

6. The positive control particle according to any of the preceding claims, wherein the aminated polymer comprises a polypeptide.

7. The positive control particle according to any of the preceding claims, wherein the nucleic acid is a double-stranded nucleic acid.

8. The positive control particle according to any of the preceding claims, wherein the nucleic acid is a deoxyribonucleic acid (DNA).

9. The positive control particle according to any of the preceding claims, wherein the nucleic acid has size ranging from 450 to 550 base pairs.

10. The positive control particle according to any of the preceding claims, wherein the aminated polymer and the nucleic acid are covalently bound directly or through a linker to a surface of the core.

11. The positive control particle according to any of the preceding claims, wherein the particle is a bead.

12. A compensation control composition comprising a plurality of positive control particles according to any of the preceding claims.

13. The compensation control composition according to claim 12, further comprising a plurality of negative control particles.

14. A labeled compensation control composition comprising a plurality of positive control particles according to any of claims 1 to 13, wherein the positive control particles have an amine reactive dye and/or a DNA binding dye bound thereto.

15. A method of determining a compensation value for cell viability data obtained from a flow cytometry analysis, the method comprising:
analyzing a labeled compensation control composition according to Claim 14 using a flow cytometer to obtain flow cytometry data; and
calculating a compensation value based on the flow cytometry data.
